# EUROPEAN PATENT APPLICATION

(11) **EP 4 147 711 A1**
(43) Date of publication of application: **15.03.2023**
(21) Application number: 21791962.0
(22) Date of filing: 19.04.2021
(51) Int. Cl.: A61K 38/16, A61K 9/08, A61K 9/19, A61K 47/02, A61K 47/04, A61K 47/12, A61K 47/26, A61P 43/00, C12N 15/12

(54) **FORMULATION CONTAINING HMGB1 PARTIAL PEPTIDE**

(30) Priority: 20.04.2020 JP 2020074929
(71) Applicant: Shionogi & Co., Ltd, Osaka-shi, Osaka 541-0045 (JP); StemRIM Inc., Osaka, 567-0085 (JP)
(72) Inventor: MATSUOKA Nao, Amagasaki-shi, Hyogo 660-0813 (JP); HATANO Shuichi, Amagasaki-shi, Hyogo 660-0813 (JP); KUGA Yuya, Amagasaki-shi, Hyogo 660-0813 (JP); OKABE Maki, Osaka-shi, Osaka 541-0045 (JP); YAMAZAKI Takehiko, Ibaraki-shi, Osaka 567-0085 (JP); YOKOTA Koichi, Ibaraki-shi, Osaka 567-0085 (JP)
(74) Representative: Mewburn Ellis LLP
(86) International application number: PCT/JP2021/015792
(87) International publication number: WO 2021/215375

(57) **Abstract**

A pharmaceutical composition containing the substance according to any one of the following (a) to (c), an organic acid, a metal salt of an organic acid, and a sugar and/or a sugar alcohol, wherein when the pharmaceutical composition is dissolved in distilled water for injection, a pH of the pharmaceutical composition is 3.0 to 4.5, the substance according to any one of the following (a) to (c) can be stabilized;
(a) an HMGB1 fragment peptide comprising the amino acid sequence set forth in SEQ ID NO: 1 or an acid addition salt thereof;
(b) a peptide comprising an amino acid sequence having substitution, deletion, insertion or addition of one or more amino acids in the amino acid sequence set forth in SEQ ID NO: 1, and having an activity of stimulating migration of a cell, or an acid addition salt thereof; and
(c) a peptide comprising an amino acid sequence having about 80% or more of sequence identity to the amino acid sequence set forth in SEQ ID NO: 1, and having an activity of stimulating migration of a cell, or an acid addition salt thereof.

## Description

### [TECHNICAL FIELD]

The present invention relates to pharmaceutical compositions for stabilizing HMGB1 partial peptides inducing tissue regeneration.

### [BACKGROUND ART]

Currently, regenerative medicine in which bone marrow mesenchymal stem cells are collected by bone marrow blood collection, expanded by cell culture, and then transplanted into a site of intractable tissue damage or peripheral circulating blood to induce regeneration of damaged tissue is actively developed. Clinical application of bone marrow mesenchymal stem cell transplantation has already been advanced in regenerative medicine such as cerebral infarction, myocardial infarction, and intractable skin ulcers. Furthermore, it has been revealed that the transplanted bone marrow mesenchymal stem cells exhibit an inhibitory action on inflammation/immune reaction and an inhibitory action on fibrous scar formation in a local place in vivo, and clinical trials of bone marrow mesenchymal stem cell transplantation therapy have been started as a new treatment method for graft-versus-host reaction (graft versus host disease, GVHD) which is a serious side effect after bone marrow transplantation or blood transfusion, and scleroderma which is an autoimmune disease. However, bone marrow blood containing bone marrow mesenchymal stem cells is collected only by invasive procedures in which a large needle is inserted into the ilium many times. In addition, bone marrow mesenchymal stem cells gradually lose their proliferation capability and pluripotency when they are continuously subcultured in vitro. Furthermore, bone marrow mesenchymal stem cell culture based on high quality control that ensures the safety of in vivo transplantation requires special culture equipment such as a cell processing center (CPC), and thus can be carried out only in extremely limited universities and companies at present. That is, in order to deliver regenerative medicine using bone marrow mesenchymal stem cells to many patients suffering from refractory tissue damage in the world, it is an urgent problem to develop a technology for mesenchymal stem cell regenerative medicine that can be implemented in any medical facility.

HMGB1 (High mobility group box 1: high mobility group 1 protein) was identified about 30 years ago as a non-histone chromatin protein that controls nuclear chromatin structure to control gene expression and DNA repair. The structure of HMGB1 protein is mainly composed of two DNA binding domains, and the DNA binding domain on the N-terminal side is referred to as A-box, and the DNA binding domain on the C-terminal side is referred to as B-box. Previous studies have revealed that a domain that binds TLR in the HMGB1 molecule and evokes an inflammatory reaction exists in the B-box.

It has been found that a fragment peptide of HMGB1 (High mobility group box 1) protein recruits bone marrow mesenchymal stem cells from bone marrow to peripheral blood, and that by administering the fragment peptide in an acute phase of myocardial infarction, bone marrow-derived mesenchymal stem cells accumulate at an infarct site and in the vicinity thereof, resulting in an effect of improving cardiac function (Patent Documents 1 and 2).

Many peptides and proteins are degraded by oral administration and are often administered as an injection. Various additives are added to the injection in order to stabilize the active ingredient, and it is known that the type of the additives greatly varies depending on the active ingredient (Patent Documents 3 to 5). Additives for stabilizing the fragment peptide of HMGB1 also had to be variously studied.

### [PRIOR ART REFERENCES]

### [Patent Document]

[Patent Document 1] International Publication brochure of WO 2012/147470
[Patent Document 2] International Publication brochure of WO 2014/065347
[Patent Document 3] International Publication brochure of WO 2008/102849
[Patent Document 4] International Publication brochure of WO 2013/154045
[Patent Document 5] JP 2000-169372 A

### [SUMMARY OF THE INVENTION]

### [PROBLEMS TO BE SOLVED BY THE INVENTION]

The purpose of the present application is to provide a pharmaceutical composition that stabilizes fragment peptides of HMGB1.

### [MEANS FOR SOLVING THE PROBLEM]

As a result of examining physical properties of additives and formulations of a pharmaceutical composition that stabilizes a fragment peptide of HMGB1, the present inventors have found that a pharmaceutical composition containing a fragment peptide of HMGB1, wherein the pH when the pharmaceutical composition is dissolved in purified water is 3 to 4.5, the pharmaceutical composition can stabilize the fragment peptide of HMGB1. Specifically, the peptide can be stabilized by containing a fragment peptide of HMGB1, a buffer of an organic acid, a saccharide and/or a sugar alcohol, and a hydroxide of an alkali metal.

That is, the present invention relates to:
(1) A pharmaceutical composition comprising the substance according to any one of the following (a) to (c), wherein the pH when the pharmaceutical composition is dissolved in distilled water for injection is 3.0 to 4.5;
   (a) an HMGB1 fragment peptide comprising the amino acid sequence set forth in SEQ ID NO: 1 or an acid addition salt thereof;
   (b) a peptide comprising an amino acid sequence having substitution, deletion, insertion or addition of one or more amino acids in the amino acid sequence set forth in SEQ ID NO: 1, and having an activity of stimulating migration of a cell, or an acid addition salt thereof; and
   (c) a peptide comprising an amino acid sequence having about 80% or more of sequence identity to the amino acid sequence set forth in SEQ ID NO: 1, and having an activity of stimulating migration of a cell, or an acid addition salt thereof,
(2) The pharmaceutical composition according to (1), comprising one or more selected from the group consisting of an organic acid, an alkali metal salt of an organic acid, and an alkaline earth metal salt of an organic acid,
(3) The pharmaceutical composition according to (2), comprising an organic acid, wherein the organic acid is one or more selected from the group consisting of citric acid, acetic acid, and phosphoric acid,
(4) The pharmaceutical composition according to (2), comprising an organic acid and/or an alkali metal salt of the organic acid, wherein the organic acid and the alkali metal salt of the organic acid are citric acid and sodium citrate, respectively,
(5) The pharmaceutical composition according to any one of (1) to (4), comprising a sugar and/or a sugar alcohol,
(6) The pharmaceutical composition according to (5), comprising a sugar, wherein the sugar is one or more selected from the group consisting of a monosaccharide, a disaccharide, and a polysaccharide,
(7) The pharmaceutical composition according to (5), wherein the sugar and/or sugar alcohol is one or more selected from the group consisting of glucose, fructose, sucrose, mannitol, and trehalose,
(8) The pharmaceutical composition according to (7), wherein the sugar and/or sugar alcohol is sucrose,
(9) The pharmaceutical composition according to any of (5) to (8), wherein the amount of sugar and/or sugar alcohol in the pharmaceutical composition is 10 to 300 times the amount of the substance according to any of (a) to (c) by mole,
(10) The pharmaceutical composition according to (9), wherein the amount of the sugar and/or sugar alcohol in the pharmaceutical composition is 50 to 300 times the amount of the substance according to any one of (a) to (c) by mole,
(11) The pharmaceutical composition according to any one of (1) to (10), wherein the pH is adjusted using a hydroxide of one or more metals selected from the group consisting of an alkali metal, an alkaline earth metal, and magnesium,
(12) The pharmaceutical composition according to (11), wherein the hydroxide is one or more selected from the group consisting of potassium hydroxide, calcium hydroxide, sodium hydroxide, and magnesium hydroxide,
(13) The pharmaceutical composition of (11), wherein the hydroxide is sodium hydroxide,
(14) The pharmaceutical composition according to any one of (1) to (13), comprising the acid addition salt according to any one of (a) to (c), wherein the acid addition salt is a trifluoroacetate salt,
(15) The pharmaceutical composition of any of (1) to (14), wherein the pharmaceutical composition is a freeze-dried product,
(16) The pharmaceutical composition according to (15), wherein the amount of the substance according to any one of (a) to (c) based on 1 g of the freeze-dried product is 6.0 to 50.0 mg,
(17) An injectable formulation prepared by dissolving a pharmaceutical composition according to any of claims (1) to (16) in distilled water for injection,
(18) A method for producing a pharmaceutical composition, comprising the steps of:
   1) dissolving a hydrate of an organic acid, a hydrate of a metal salt of an organic acid, and a sugar and/or a sugar alcohol in distilled water for injection to adjust the pH to 3.0 to 4.0;
   2) cooling a liquid produced in the step 1) to 18°C or lower;
   3) dissolving the substance according to any one of (a) to (c) of claim 1 in the liquid obtained in the step 2);
   4) adjusting a pH of a liquid obtained in the step 3) to 2.5 to 4.0 with a hydroxide of one or more metals selected from the group consisting of an alkali metal, an alkaline earth metal, and magnesium; and
   5) freeze-drying a liquid obtained in the step 4),
(19) A method for producing a pharmaceutical composition, comprising the steps of:
   1) dissolving citric acid hydrate, sodium citrate hydrate and sucrose in distilled water for injection and adjusting the pH to 3.0 to 4.0;
   2) cooling a liquid produced in the step 1) to 18°C or lower;
   3) dissolving the substance according to any one of (a) to (c) of claim 1 in the liquid obtained in the step 2);
   4) adjusting a pH of the liquid obtained in the step 3) to 2.9 to 3.5 with sodium hydroxide; and
   5) freeze-drying a liquid obtained in the step 4),
(20) A pharmaceutical composition produced by the production method according to (18) or (19).

### [EFFECT OF THE INVENTION]

The pharmaceutical composition containing the HMGB1 fragment peptide of the present invention is a pharmaceutical composition capable of enhancing stability by pH or an additive.

### [MODE FOR CARRYING OUT THE INVENTION]

The active ingredients of the formulations of the present invention are as defined herein:
(a) an HMGB1 fragment peptide comprising the amino acid sequence set forth in SEQ ID NO: 1 or an acid addition salt thereof;
(b) a peptide comprising an amino acid sequence having substitution, deletion, insertion or addition of one or more amino acids in the amino acid sequence set forth in SEQ ID NO: 1, and having an activity of stimulating migration of a cell, or an acid addition salt thereof; or
(c) a peptide comprising an amino acid sequence having about 80% or more of sequence identity to the amino acid sequence set forth in SEQ ID NO: 1, and having an activity of stimulating migration of a cell, or an acid addition salt thereof.

In the present application, the term "pharmaceutical composition" is used interchangeably with "medicine", "drug" or "pharmaceutical composition".

In the present application, the HMGB1 fragment peptide comprising the amino acid sequence set forth in SEQ ID NO: 1 means a peptide consisting of a part of HMGB1 protein and comprising the amino acid sequence set forth in SEQ ID NO: 1. Such a peptide can be obtained as a recombinant by incorporating a DNA encoding the peptide into an appropriate expression system, or can be artificially synthesized.

In the present application, examples of the HMGB1 protein include, but are not limited to, a protein comprising the amino acid sequence set forth in SEQ ID NO: 2 and a protein encoded by DNA comprising the base sequence set forth in SEQ ID NO: 3.

Examples of the HMGB1 fragment peptide comprising the amino acid sequence set forth in SEQ ID NO: 1 in the present application include, but are not limited to:
1) an HMGB1 fragment peptide comprising the amino acid sequence set forth in SEQ ID NO: 1 and having an activity of stimulating migration of a cell;
2) an HMGB1 fragment peptide comprising the amino acid sequence set forth in SEQ ID NO: 1 and having an activity of stimulating migration of mesenchymal stem cell;
3) an HMGB1 fragment peptide consisting of the amino acid sequence set forth in SEQ ID NO: 1.

In the present application, the cells whose migration is stimulated by the HMGB1 fragment peptide include, but are not limited to, bone marrow cells or bone marrow-derived cells (for example, bone marrow stem cells or bone marrow-derived stem cells).

In the present application, the "bone marrow cell" means a cell present in the bone marrow, while the "bone marrow-derived cell" means a "bone marrow cell" recruited from the bone marrow to the outside of the bone marrow. In addition, the "bone marrow cell" may include undifferentiated cells such as stem cells and precursor cells present in the bone marrow.

In the present application, examples of cells whose migration is stimulated by the HMGB1 fragment peptide include, but are not limited to, a mesenchymal stem cell. The "mesenchymal stem cell " is a cell that is collected from bone marrow or other tissues (blood, for example, umbilical cord blood, and skin, fat, dental pulp, etc.), can be cultured and proliferated as an adherent cell to a culture dish (made of plastic or glass), and has the ability to differentiate into mesenchymal tissues such as bone, cartilage, fat, and muscle. In one aspect, the mesenchymal stem cells also have the ability to differentiate into epithelial tissue and neural tissue. The mesenchymal stem cell in the present application may exist as a heterogeneous cell population including not only narrow-sense a stem cell but also a precursor cell, and may also include a differentiated cell in addition to narrow-sense a stem cell and/or a precursor cell under culture conditions. In one aspect, the mesenchymal stem cells may be composed only of stem cells in a narrow sense, or may be a cell population composed of a plurality of types of precursor cells.

In the present application, the precursor cell is defined as a cell having unidirectional differentiation ability into a specific tissue cell other than the blood system, and include a cell having the ability to differentiate into a mesenchymal tissue, an epithelial tissue, a neural tissue, solid organ, and vascular endothelium.

In the present application, the cells whose migration is stimulated by the HMGB1 fragment peptide include, but are not limited to, a bone marrow mesenchymal stem cell and a bone marrow-derived mesenchymal stem cell. The "bone marrow mesenchymal stem cell" is a cell existing in the bone marrow, is collected from the bone marrow, can be cultured and proliferated as an adherent cell to a culture dish (made of plastic or glass), and has a characteristic of having the ability to differentiate into mesenchymal tissue such as bone, cartilage, fat, and muscle, and neural tissue and epithelial tissue. In the present application, the term "bone marrow mesenchymal stem cell" is used interchangeably with "bone marrow mesenchymal stromal cell", "bone marrow pluripotent stem cell" and "bone marrow pluripotent stromal cell".

In addition, the "bone marrow-derived mesenchymal stem cell" refers to a bone marrow mesenchymal stem cell recruited from the bone marrow to the outside of the bone marrow, and is a cell that can be obtained by peripheral blood sampling, and further sampling from a mesenchymal tissue such as fat, an epithelial tissue such as skin, and a neural tissue such as brain. In the present application, the term "bone marrow-derived mesenchymal stem cell" is used interchangeably with "bone marrow-derived mesenchymal stromal cell", "bone marrow-derived pluripotent stem cell", and "bone marrow-derived pluripotent stromal cell".

In addition, a bone marrow mesenchymal stem cell and a bone marrow-derived mesenchymal stem cell are also characterized by having the ability to differentiate into, for example, an epithelial tissue such as a keratinocyte constituting the skin and a nervous system tissue constituting the brain either directly after collection or by administering the cell attached once to a culture dish to a damaged part of a living body.

A bone marrow mesenchymal stem cell and a bone marrow-derived mesenchymal stem cell preferably have the ability to differentiate into an osteoblast (can be identified by recognizing calcium deposition when differentiation is induced), a chondrocyte (can be identified by positive Alcian blue staining, positive safranin-O staining, etc.), a fat cell (identifiable by positive Sudan III staining, etc.), as well as a fibroblast, a smooth muscle cell, a skeletal muscle cell, a stromal cell, a mesenchymal cell such as a tendon cell, a nerve cell, a pigment cell, an epidermal cell, a hair follicle cell (expressing cytokeratin family, hair keratin family, etc.), an epithelial cell (for example, an epidermal keratinocyte, an intestinal epithelial cell express cytokeratin family, etc.), an endothelial cell, in addition, solid organ cells such as liver, kidney, pancreas, but the differentiated cell is not limited to the above cells.

Examples of the human mesenchymal stem cell marker include, but are not limited to, all or a part of PDGFRα positive, PDGFRβ positive, Lin negative, CD 45 negative, CD 44 positive, CD 90 positive, CD 29 positive, Flk-1 negative, CD 105 positive, CD 73 positive, CD 90 positive, CD 71 positive, Stro-1 positive, CD 106 positive, CD 166 positive, CD 31 negative, CD 271 positive, and CD 11b negative.

Examples of the mouse mesenchymal stem cell marker used in a mouse include, but are not limited to, all or a part of CD 44 positive, PDGFRα positive, PDGFRβ positive, CD 45 negative, Lin negative, Sca-1 positive, c-kit negative, CD 90 positive, CD 105 positive, CD 29 positive, Flk-1 negative, CD 271 positive, and CD 11b negative.

Examples of the rat mesenchymal stem cell marker used for rats include, but are not limited to, all or a part of PDGFRα positive, CD 44 positive, CD 54 positive, CD 73 positive, CD 90 positive, CD 105 positive, CD 29 positive, CD 271 positive, CD 31 negative, and CD 45 negative.

In the present application, examples of cell whose migration is stimulated by the HMGB1 fragment peptide also include, but are not limited to, PDGFRα-positive cells. Examples of the PDGFRα-positive cell whose migration is stimulated by the HMGB1 fragment peptide include, but are not limited to, a PDGFRα-positive mesenchymal stem cell, a PDGFRα-positive bone marrow-derived mesenchymal stem cell, and a PDGFRα-positive bone marrow-derived cell, which are obtained as an adherent cell by mononuclear fractionated cell culture in blood obtained by bone marrow collection (bone marrow cell collection) or peripheral blood collection. Examples of the PDGFRα-positive mesenchymal stem cell include a cell positive for PDGFRα and CD 44, a cell positive for PDGFRα and CD 90, a cell positive for PDGFRα and CD 105, a cell positive for PDGFRα and CD 29, and the like. In one aspect, the PDGFRα-positive mesenchymal stem cell may be a cell negative for CD 44.

In the formulation of the present invention, in place of or in addition to the HMGB1 fragment peptide comprising the amino acid sequence set forth in SEQ ID NO: 1, a peptide comprising an amino acid sequence in which one or more amino acid residues are modified (having substitution, deletion, insertion, or addition) in the amino acid sequence set forth in SEQ ID NO: 1 and having an activity of stimulating migration of a cell can also be used. Examples of such a peptide may include, but are not limited to:
i) a peptide comprising an amino acid sequence having substitution, deletion, insertion or addition of one or more (for example, 1 to 10, 1 to 9, 1 to 8, 1 to 7, 1 to 6, 1 to 5, 1 to 4, 1 to 3, or 1 or 2) amino acids in the amino acid sequence set forth in SEQ ID NO: 1, and having an activity of stimulating migration of a cell;
ii) a peptide consisting of an amino acid sequence having substitution, deletion, insertion or addition of one or more (for example, 1 to 10, 1 to 9, 1 to 8, 1 to 7, 1 to 6, 1 to 5, 1 to 4, 1 to 3, or 1 or 2) amino acids in the amino acid sequence set forth in SEQ ID NO: 1, and having an activity of stimulating migration of a cell;
iii) a peptide comprising an amino acid sequence having about 80% or more, for example, about 85% or more, about 90% or more, about 91% or more, about 92% or more, about 93% or more, about 94% or more, about 95% or more, about 96% or more, about 97% or more, about 98% or more, or about 99% or more of sequence identity to the amino acid sequence set forth in SEQ ID NO: 1, and having an activity of stimulating migration of a cell; and
iv) A peptide comprising an amino acid sequence having about 80% or more, for example, about 85% or more, about 90% or more, about 91% or more, about 92% or more, about 93% or more, about 94% or more, about 95% or more, about 96% or more, about 97% or more, about 98% or more, or about 99% or more of sequence identity to the amino acid sequence set forth in SEQ ID NO: 1, and having an activity of stimulating migration of a cell.

In addition, examples of the cell whose migration is stimulated by these peptides include, but are not limited to, a mesenchymal stem cell, a bone marrow-derived mesenchymal stem cell, a PDGFRα-positive cell, a PDGFRu-positive mesenchymal stem cell, a PDGFRα-positive bone marrow-derived mesenchymal stem cell, and a PDGFRα-positive bone marrow-derived cell, which are obtained as an adherent cell by mononuclear fractionated cell culture in blood obtained by bone marrow collection (bone marrow cell collection) or peripheral blood collection.

The HMGB1 fragment peptide in the formulation of the present invention may be a salt, but is preferably an acid addition salt. The acid addition salt is, for example, a salt to which an inorganic acid such as sulfuric acid or hydrochloric acid, or an organic acid such as trifluoroacetic acid or p-toluenesulfonic acid is added. Preferred acid addition salt is trifluoroacetate salt.

An effective amount of a formulation of the invention containing a peptide of the present application is administered to a subject for the treatment or prevention of a disease or condition described herein.

In particular, the HMGB1 fragment peptide, which is an active ingredient of the formulation of the present invention, is effective for epidermolysis bullosa, cerebral infarction, myocardial infarction, heart disease, old myocardial infarction, spinal cord injury, fibrous disease, inflammatory bowel disease, psoriasis, and the like.

The effective amount in the present application refers to an amount sufficient for treating or preventing a disease or condition described in the present specification. The treatment in the present application includes, but is not limited to, reduction, delay, inhibition, improvement, remission, cure, complete cure, and the like. In addition, the prevention in the present application includes, but is not limited to, reduction, delay, inhibition, and the like.

The subject in the present application is not particularly limited, and examples thereof include mammals, birds, and fish. Examples of the mammal include a human or a non-human animal, and examples thereof include, but are not limited to, a human, a mouse, a rat, a monkey, a pig, a dog, a rabbit, a hamster, a guinea pig, a horse, a sheep, and a whale. In the present application, the term "subject" is used interchangeably with "patient", "individual", and "animal".

The administration site of the peptide or the like of the present application is not limited, and the peptide or the like of the present application can exert its effect even when it is administered to any site such as a site having a structural or functional abnormality in a tissue or the vicinity thereof, a site different from these sites (other sites), a site away from a site having a structural or functional abnormality in a tissue, a site distal to a site having a structural or functional abnormality in a tissue, or a site distal and ectopic to a site having a structural or functional abnormality in a tissue.

Examples of the administration method of the formulation of the present invention include, but are not limited to, oral administration and parenteral administration, and examples of the parenteral administration method include intravascular administration (intra-arterial administration, intravenous administration, etc.), intramuscular administration, subcutaneous administration, intradermal administration, intraperitoneal administration, nasal administration, pulmonary administration, and transdermal administration. In addition, the peptide or the like of the present application can be systemically or locally (for example, subcutaneous, intradermal, skin surface, ocular or palpebral conjunctiva, nasal mucous membrane, intraoral and gastrointestinal mucous membrane, vaginal/intrauterine mucous membrane, damaged site, or the like) administered by injection administration, for example, intravenous injection, intramuscular injection, intraperitoneal injection, subcutaneous injection, or the like.

The formulation of the present invention can be administered by intravenous drip infusion. In the case of intravenous drip infusion, the freeze-dried formulation in the vial can be redissolved in physiological saline, and the resulting solution can be added to a physiological saline bag to form an administration solution. The concentration of this administration solution is 0.1 to 2.5 mg/mL, and can be administered by intravenous drip infusion.

In addition, in place of the peptide or the like of the present application, a cell that secretes the peptide of the present application, a vector for gene therapy into which a DNA encoding the peptide is inserted, and a pharmaceutical composition containing them can also be used.

In addition, the administration method can be appropriately selected depending on the age and condition of the patient. When the peptide of the present application is administered, for example, the dose can be selected in the range of 0.0000001 mg to 1000 mg per 1 kg of body weight per administration. When a cell that secretes the peptide of the present application or a vector for gene therapy into which a DNA encoding the peptide is inserted is administered, the peptide can be administered so that the amount of the peptide is within the above range. However, the pharmaceutical composition in the present application is not limited to these doses.

The content of the HMGB1 fragment peptide or the acid addition salt thereof in the formulation of the present invention is not particularly limited, but may be any content (titer) as long as the HMGB1 fragment peptide or the acid addition salt thereof can be stabilized in the formulation. The content of the HMGB1 fragment peptide or acid addition salt thereof in the formulation of the present invention is usually 1.0 to 50.0 wt%, preferably 3.0 to 40.0 wt%, more preferably 6.0 to 30.0 wt%, and particularly preferably 6.5 to 28.3 wt% (64.6 to 283.3 mg/g) with respect to the total amount of the formulation, particularly when the formulation is a freeze-dried formulation. If the content is less than this amount, the formulation may have to be overdosed, and if it is more, it may not be freeze-dried.

When the formulation of the present invention is dissolved in distilled water for injection, the pH is 3.0 to 4.5, preferably 3.0 to 4.3, more preferably 3.0 to 4.0, and particularly preferably 3.0 to 3.6. The pH is measured by a pH meter or the like, but an error of about ± 0.05 may occur during the measurement.

The formulation of the present invention can contain various additives.

In the formulation of the present invention, a buffer may be contained, and buffers described in Japanese Pharmacopoeia, Japanese Pharmaceutical Codex, Japanese Pharmaceutical Excipients, Japan's Specifications and Standards for Food Additives, or the like can be used. A buffer is a compound added to a solution in order to keep the pH of the solution at a specific value even when another acid or base is added to the solution to some extent. That is, the buffer suppresses a rapid change in pH. Examples of the buffer include an amino acid or salts such as hydrochloride, sodium salt, and potassium salt thereof, a salt such as an amphoteric electrolyte or a sodium salt thereof, salts such as sodium and potassium salts of a weak acid, a combination of a salt of a weak acid with weak acids, a strong acid such as hydrochloric acid, or sulfuric acid, and trishydroxymethylaminomethane. Examples of the amino acid or salts such as hydrochloride, sodium salt, and potassium salt thereof include amino acids such as glycine, alanine, arginine, glutamine, cysteine, serine, threonine, valine, histidine, phenylalanine, methionine, aspartic acid, glutamic acid, ε-aminocaproic acid, lysine, and leucine, or salts such as hydrochloride, sodium salt, and potassium salt thereof. Examples of the amphoteric electrolyte or a salt such as a sodium salt thereof include amphoteric electrolytes such as nicotinamide and aminobenzoic acid, and salts such as a sodium salt thereof. Examples of salts such as sodium salts and potassium salts of weak acids include sodium salts and potassium salts of weak acids such as citric acid, phosphoric acid, lactic acid, acetic acid, boric acid, propionic acid, butyric acid, valeric acid, glycolic acid, oxalic acid, malonic acid, succinic acid, glutaric acid, adipic acid, malic acid, fumaric acid, maleic acid, benzoic acid, salicylic acid, phthalic acid, and tartaric acid. Examples of the combination of a weak acid or a salt thereof and a strong acid such as hydrochloric acid or sulfuric acid include a combination of a salt of a weak acid such as citric acid, phosphoric acid, lactic acid, acetic acid, boric acid, propionic acid, butyric acid, valeric acid, glycolic acid, oxalic acid, malonic acid, succinic acid, glutaric acid, adipic acid, malic acid, fumaric acid, maleic acid, benzoic acid, salicylic acid, phthalic acid or tartaric acid and a strong acid such as a weak acid, hydrochloric acid or sulfuric acid. Citric acid, sodium citrate, citric acid hydrate, sodium citrate hydrate, sodium dihydrogen citrate, disodium citrate, calcium citrate, acetic acid, sodium acetate, sodium acetate hydrate, phosphoric acid, calcium monohydrogen phosphate, dipotassium phosphate, monopotassium phosphate, disodium phosphate, monosodium phosphate are preferable. Citric acid, sodium citrate, citric acid hydrate, and sodium citrate hydrate are more preferable. Citric acid hydrate and sodium citrate hydrate are particularly preferable. Two or more types of these buffers may be used.

The content of the buffer in the formulation of the present invention is not particularly limited, but may be any content as long as the HMGB1 fragment peptide or the acid addition salt thereof can be stabilized in the formulation. The content of the buffer in the formulation of the present invention is usually 0.1 to 10 wt%, preferably 0.25 to 7.5 wt%, more preferably 0.5 to 5.0 wt%, and particularly preferably 0.9 to 3.9 wt% with respect to the total amount of the formulation, particularly when the formulation is a freeze-dried formulation. There is a risk that the HMGB1 fragment peptide as an active ingredient is not stabilized even if the content is less than or more than this amount.

The formulation of the present invention may contain an additive that stabilizes the HMGB1 fragment peptide as an active ingredient or an acid addition salt thereof (Hereinafter, the term "stabilizer" may be used in the present specification.), and additives listed in Japanese Pharmacopoeia, Japanese Pharmaceutical Codex, Japanese Pharmaceutical Excipients, Japan's Specifications and Standards for Food Additives, or the like can be used. Examples of the additive for stabilizing the active ingredient include a saccharide, a sugar alcohol, an amino acid, an alkali metal chloride, and an alkaline earth metal chloride.

Examples of the saccharide include a monosaccharide, a disaccharide, and a polysaccharide, and specific examples thereof include sucrose (white sugar, refined white sugar), trehalose, glucose, fructose, raffinose, maltose, and maltotriose sucrose.

Examples of the sugar alcohol include mannitol, sorbitol, erythritol, xylitol, powdered maltose syrup, and maltitol.

Examples of the amino acid include methionine, histidine, and arginine.

Examples of the alkali metal chloride include sodium chloride, lithium chloride, and potassium chloride.

Examples of the alkaline earth metal chloride include barium chloride and calcium chloride.

A saccharide and a sugar alcohol are preferable, and a monosaccharide, a disaccharide, a polysaccharide and the like are more preferable. In addition, glucose, fructose, sucrose, mannitol, and trehalose are more preferable, and sucrose (white sugar, refined white sugar) is particularly preferable.

The content of the stabilizer in the formulation of the present invention is not particularly limited, but may be any content as long as the HMGB1 fragment peptide or the acid addition salt thereof can be stabilized in the formulation. The content of the stabilizer in the formulation of the present invention is usually 30.0 to 99.0 wt%, preferably 35.0 to 97.5 wt%, more preferably 40.0 to 95.0 wt%, and particularly preferably 67.8 to 92.7 wt% with respect to the total amount of the formulation, particularly when the formulation is a freeze-dried formulation. There is a risk that the HMGB1 fragment peptide as an active ingredient is not stabilized even if the content is more than or less than this amount.

The content of the stabilizer in the formulation of the present invention, particularly the amount of the sugar and/or sugar alcohol is 10 to 300 times the amount of the HMGB1 fragment peptide as the active ingredient by mole, preferably 25 to 300 times by mole, and more preferably 50 to 300 times by mole. even if the content is more than or less than this amount, it may not be possible to produce a freeze-dried formulation.

The formulation of the present invention is produced by freeze-drying a liquid (Hereinafter, the liquid may be referred to as a "preparation solution" in the present specification.) containing HMGB1 fragment peptide as an active ingredient or an acid addition salt thereof, or an additive to produce a freeze-dried formulation. Depending on the pH of this preparation solution, the stability of the HMGB1 fragment peptide in the freeze-dried formulation changes. When the HMGB1 fragment peptide is stabilized, the pH of this preparation solution is pH 2.5 to 5.0, preferably pH 2.5 to 4.5, more preferably pH 2.5 to 4.0, and particularly preferably pH 2.9 to 3.5. The concentration of the active ingredient in the preparation solution may be any concentration that can be freeze-dried, and is usually 1.0 to 100.0 mg/g, preferably 2.5 to 80.0 mg/g, more preferably 5.0 to 60.0 mg/g, and particularly preferably 7.5 to 50.0 mg/g.

As described above, it is necessary to adjust the pH of the preparation solution of the formulation of the present invention, and the pH can be adjusted by an additive capable of adjusting the pH of the preparation solution. For example, the pH can be adjusted by an alkali metal hydroxide, an alkaline earth metal hydroxide, or a magnesium hydroxide. Potassium hydroxide, calcium hydroxide, sodium hydroxide, magnesium hydroxide can be preferably used, and sodium hydroxide can be more preferably used.

The amount of the additive for adjusting the pH of the preparation solution of the formulation of the present invention may be any amount as long as it can adjust the optimum pH of the preparation solution.

In the formulation of the present invention, as other additives, the additives generally used for injections may be added, and the additives listed in Japanese Pharmacopoeia, Japanese Pharmaceutical Codex, Japanese Pharmaceutical Excipients or the like, Japan's Specifications and Standards for Food Additives, or the like may be added. Examples of the additive include a suspending agent, a surfactant, a hydrophilic polymer, an emulsifier, a preservative, a soothing agent, a tonicity agent, and a solvent.

The formulation of the present invention may contain a suspending agent. The suspending agent may be any additive as long as it can suspend the active ingredient in a liquid, and the suspending agents listed in Japanese Pharmacopoeia, Japanese Pharmaceutical Codex, Japanese Pharmaceutical Excipients or the like, Japan's Specifications and Standards for Food Additives, or the like can be used. Examples of the suspending agent include a surfactant, a hydrophilic polymer, gum arabic, and tragacanth. Examples of the surfactant include stearyl triethanolamine, sodium lauryl sulfate, lauryl aminopropionate, lecithin, benzalkonium chloride, benzethonium chloride, glyceryl monostearate, poloxamer, aluminum monostearate, and polysorbate 80. Examples of the hydrophilic polymer include polyvinyl alcohol, polyvinyl pyrrolidone, carboxymethylcellulose sodium, methyl cellulose, hydroxymethyl cellulose, hydroxyethyl cellulose, and hydroxypropyl cellulose.

The formulation of the present invention may contain an emulsifier. The suspending agent may be any emulsifiers as long as it can emulsify the active ingredient in a liquid, and the emulsifiers listed in Japanese Pharmacopoeia, Japanese Pharmaceutical Codex, Japanese Pharmaceutical Excipients or the like, Japan's Specifications and Standards for Food Additives, or the like can be used. Examples of the emulsifier include a phospholipid, a surfactant, colloidal clay (colloidal clay), a metal hydroxide, natural gum, and gelatin. Examples of the phospholipid include a natural phospholipid and a synthetic phospholipid (an anionic phospholipid). Examples of the natural phospholipid include egg yolk lecithin, soybean lecithin, soybean, and lecithin. Examples of the synthetic phospholipid (anionic phospholipid) include dimyristoylphosphatidylglycerol, dipalmitoylphosphatidylglycerol, distearoylphosphatidyl glycerol, dioleoylphosphatidylglycerol, oleoylpalmitoylphosphatidylglycerol, dioctanoylphosphatidicacid, didecanoylphosphatidicacid, dilauroylphosphatidicacid, dimyristoylphosphatidicacid, dipalmitoylphosphatidicacid, diheptadecanoylphosphatidicacid, distearoylphosphatidicacid, dioleoylphosphatidicacid, arachidonylstearoylphosphatidicacid, dipalmitoylphosphatidylserine, dioleoylphosphatidylserine, dimyristoylphosphatidylinositol, dipalmitoylphosphatidylphosphatidyl, distearoylphosphatidylinositol, dioleoylphosphatidylinositol, dimyristoylphosphatidylserine, and distearoylphosphatidylserine. Examples of the surfactant include a nonionic surfactant, an anionic surfactant, and a cationic surfactant. Examples of the nonionic surfactant include a polyoxyethylenepolyoxypropylene copolymer, a polyoxyethylene alkyl ether, a polyoxyethylene alkyl aryl ether, a hydrogenated castor oil polyoxyethylene derivative, a polyoxyethylene sorbitan derivative, a polyoxyethylene sorbitol derivative, a polyoxyethylene alkyl ether sulfate, a polyoxyethylene sorbitan fatty acid ester, a polyoxyethylene castor oil derivative, polyvinylpyrrolidone, polyvinyl alcohol, carboxymethyl cellulose, lecithin, gelatin, hyaluronic acid, a sucrose fatty acid ester, a glycerin fatty acid ester, a sorbitan fatty acid ester, and a propylene glycol fatty acid ester. Examples of the anionic surfactant include sodium oleate, sodium stearate, calcium stearate, and sodium lauryl sulfate. Examples of the cationic surfactant include benzalkonium chloride. Examples of the colloidal clay (colloidal clay) include bentonite and VEEGUM. Examples of the metal hydroxide include magnesium hydroxide, aluminum hydroxide, potassium hydroxide, and sodium hydroxide. Examples of the natural gum include gum arabic, gum tragacanth, and tragacanth.

The formulation of the present invention may contain a preservative. As the preservative, preservatives described in Japanese Pharmacopoeia, Japanese Pharmaceutical Codex, Japanese Pharmaceutical Excipients, Japan's Specifications and Standards for Food Additives, or the like can be used. Examples of the preservative include parabens, benzoic acids, sorbic acid, potassium sorbate, sodium dehydroacetate, benzyl alcohol, chlorobutanol, cresol, sodium dehydroacetate, and sodium edetate. Examples of the parabens include methylparaben, ethylparaben, propylparaben, butylparaben, and the like. Examples of the benzoic acids include benzoic acid, sodium benzoate, benzyl benzoate, and paraoxybenzoic acid esters (isobutyl parahydroxybenzoate, isopropyl parahydroxybenzoate, ethyl parahydroxybenzoate, ethyl sodium parahydroxybenzoate, butyl parahydroxybenzoate, propyl parahydroxybenzoate, propyl sodium parahydroxybenzoate, methyl parahydroxybenzoate, methyl sodium parahydroxybenzoate).

The formulation of the present invention may contain a soothing agent. As the soothing agent, soothing agents described in Japanese Pharmacopoeia, Japanese Pharmaceutical Codex, Japanese Pharmaceutical Excipients, Japan's Specifications and Standards for Food Additives, or the like can be used. Examples of the soothing agent include ethyl aminobenzoate, inositol, liquid phenol, meprylcaine hydrochloride, lidocaine hydrochloride, creatinine, chlorobutanol, sodium hydrogen carbonate, clove oil, glucose, procaine hydrochloride, propylene glycol, benzyl alcohol, mepivacaine hydrochloride, lidocaine, magnesium sulfate hydrate, and benzalkonium chloride.

The formulation of the present invention may contain an antiseptic. As the antiseptic, antiseptics described in Japanese Pharmacopoeia, Japanese Pharmaceutical Codex, Japanese Pharmaceutical Excipients, Japan's Specifications and Standards for Food Additives, or the like can be used. Examples of the antiseptic include paraoxybenzoic acid esters (paraben), alcohol, benzalkonium chloride, benzoic acid, salicylic acid, sorbic acid, chlorhexidine, and hydrogen peroxide. Examples of the paraoxybenzoic acid esters (paraben) include methyl paraoxybenzoate (methylparaben) and propyl paraoxybenzoate (propylparaben). Examples of the alcohol include benzyl alcohol, phenethyl alcohol, chlorobutanol, and phenol.

The formulation of the present invention may contain a tonicity agent. As the tonicity agent, tonicity agents described in Japanese Pharmacopoeia, Japanese Pharmaceutical Codex, Japanese Pharmaceutical Excipients, Japan's Specifications and Standards for Food Additives, or the like can be used. Examples of the tonicity agent include an ionic tonicity agent and a nonionic tonicity agent. Examples of the ionic tonicity agent include inorganic salts and organic bases. Examples of the inorganic salts include disodium hydrogen phosphate, sodium dihydrogen phosphate, potassium dihydrogen phosphate, sodium hydrogen sulfite, sodium sulfite, sodium thiosulfate, magnesium sulfate, sodium chloride, potassium chloride, calcium chloride, magnesium chloride, boric acid, and borax. Examples of the organic bases include potassium acetate, sodium acetate, sodium hydrogen carbonate, and sodium carbonate. Examples of the nonionic tonicity agent include a polyhydric alcohol having two or more alcoholic hydroxyl groups in the molecule, an amino acid, and a saccharide. Examples of the polyhydric alcohol having two or more alcoholic hydroxyl groups in the molecule include glycerin, propylene glycol, and polyethylene glycol. Examples of the amino acid include glycine and alanine. Examples of the saccharide include glucose, fructose, trehalose, sucrose, xylitol, sorbitol, and mannitol.

The formulation of the present invention may contain a solvent. In addition, the formulation of the present invention may be dissolved or suspended in a solvent. As the solvent, solvents described in Japanese Pharmacopoeia, Japanese Pharmaceutical Codex, Japanese Pharmaceutical Excipients, Japan's Specifications and Standards for Food Additives, or the like can be used. Examples of the solvent include an aqueous solvent, alcohol, propylene glycol, macrogol, sesame oil, and corn oil. Examples of the aqueous solvent include distilled water, physiological saline, Ringer's solution, water for injection, and distilled water for injection.

The dosage form of the formulation of the present invention may be any dosage form described in each section of the formulation of the Japanese Pharmacopoeia, but is preferably an injection. The injection is administered directly into a blood vessel or a tissue, or may be administered by drip infusion.

The production method of the formulation of the present invention may be a method for producing a pharmaceutical composition capable of stabilizing the HMGB1 fragment peptide or the acid addition salt thereof as an active ingredient. For example, a method for producing a pharmaceutical composition may include the following steps.
1) dissolving an additive containing a hydrate of an organic acid, a hydrate of an alkali metal salt of an organic acid, a sugar and/or a sugar alcohol in distilled water for injection and adjusting the pH to 3.0 to 4.0;
2) cooling a liquid produced in the step 1) to 18°C or lower;
3) dissolving the HMGB1 fragment peptide or the acid addition salt thereof in the liquid obtained in the step 2);
4) adjusting a pH of a liquid obtained in the step 3) to 2.5 to 4.0 with a hydroxide of one or more metals selected from the group consisting of an alkali metal, an alkaline earth metal, and magnesium; and
5) freeze-drying a liquid obtained in the step 4). After the step 4) and before the step 5), a step of adjusting the concentration with distilled water for injection may be included.

Another aspect of the production method of the formulation of the present invention may be a method for producing a pharmaceutical composition capable of stabilizing the HMGB1 fragment peptide as an active ingredient or a trifluoroacetate salt thereof. For example, a method for producing a pharmaceutical composition may include the following steps.
1) dissolving additives containing citric acid hydrate, sodium citrate hydrate and sucrose in distilled water for injection and adjusting the pH to 3.0 to 4.0;
2) cooling a liquid produced in the step 1) to 18°C or lower;
3) dissolving the HMGB1 fragment peptide or the trifluoroacetate salt thereof in the liquid obtained in the step 2);
4) adjusting a pH of the liquid obtained in the step 3) to 2.9 to 3.5 with sodium hydroxide; and
5) freeze-drying a liquid obtained in the step 4). After the step 4) and before the step 5), a step of adjusting the concentration with distilled water for injection may be included.

The pharmaceutical composition of the formulation of the present invention is a pharmaceutical composition containing an HMGB1 fragment peptide as an active ingredient or an acid addition salt thereof, an organic acid, a metal salt of an organic acid, and a sugar and/or a sugar alcohol.

As another aspect of the pharmaceutical composition of the formulation of the present invention, there is a pharmaceutical composition containing an HMGB1 fragment peptide as an active ingredient or an acid addition salt thereof, an organic acid, a metal salt of an organic acid, and one or more selected from the group consisting of a monosaccharide, a disaccharide, and a polysaccharide as a sugar and/or a sugar alcohol.

As another aspect of the pharmaceutical composition of the formulation of the present invention, there is a pharmaceutical composition containing an HMGB1 fragment peptide as an active ingredient or an acid addition salt thereof, an organic acid, a metal salt of an organic acid, and one or more selected from the group consisting of glucose, fructose, sucrose, mannitol, and trehalose as a sugar and/or a sugar alcohol.

As another aspect of the pharmaceutical composition of the formulation of the present invention, there is a pharmaceutical composition containing an HMGB1 fragment peptide as an active ingredient or a trifluoroacetate salt thereof, an organic acid, a metal salt of an organic acid, and sucrose.

As another aspect of the pharmaceutical composition of the formulation of the present invention, there is a pharmaceutical composition containing an HMGB1 fragment peptide as an active ingredient or a trifluoroacetate salt thereof, citric acid, a metal salt of an organic acid, and sucrose.

As another aspect of the pharmaceutical composition of the formulation of the present invention, there is a pharmaceutical composition containing an HMGB1 fragment peptide as an active ingredient or a trifluoroacetate salt thereof, an organic acid, sodium citrate, and sucrose.

As another aspect of the pharmaceutical composition of the formulation of the present invention, there is a pharmaceutical composition containing HMGB1 fragment peptide as active ingredient or a trifluoroacetate salt thereof, citric acid, sodium citrate, and sucrose.

The pharmaceutical composition of the formulation of the present invention is a pharmaceutical composition containing an HMGB1 fragment peptide as an active ingredient or an acid addition salt thereof, an organic acid, a metal salt of an organic acid, and a sugar and/or a sugar alcohol, and having a pH of 3.0 to 4.5 when the pharmaceutical composition is dissolved in distilled water for injection.

As another aspect of the pharmaceutical composition of the formulation of the present invention, there is a pharmaceutical composition containing an HMGB1 fragment peptide as an active ingredient or an acid addition salt thereof, an organic acid, a metal salt of an organic acid, and one or more selected from the group consisting of a monosaccharide, a disaccharide, and a polysaccharide as a sugar and/or a sugar alcohol, and having a pH of 3.0 to 4.5 when the pharmaceutical composition is dissolved in distilled water for injection.

As another aspect of the pharmaceutical composition of the formulation of the present invention, there is a pharmaceutical composition containing an HMGB1 fragment peptide as an active ingredient or an acid addition salt thereof, an organic acid, a metal salt of an organic acid, and one or more selected from the group consisting of glucose, fructose, sucrose, mannitol, and trehalose as a sugar and/or a sugar alcohol, and having a pH of 3.0 to 4.5 when the pharmaceutical composition is dissolved in distilled water for injection.

As another aspect of the pharmaceutical composition of the formulation of the present invention, there is a pharmaceutical composition containing HMGB1 fragment peptide as an active ingredient or a trifluoroacetate salt thereof, an organic acid, a metal salt of an organic acid, and sucrose, and having a pH of 3.0 to 4.5 when the pharmaceutical composition is dissolved in distilled water for injection.

As another aspect of the pharmaceutical composition of the formulation of the present invention, there is a pharmaceutical composition containing HMGB1 fragment peptide as an active ingredient or a trifluoroacetate salt thereof, citric acid, a metal salt of an organic acid, and sucrose, and having a pH of 3.0 to 4.5 when the pharmaceutical composition is dissolved in distilled water for injection.

As another aspect of the pharmaceutical composition of the formulation of the present invention, there is a pharmaceutical composition containing HMGB1 fragment peptide as an active ingredient or a trifluoroacetate salt thereof, an organic acid, sodium citrate, and sucrose, and having a pH of 3.0 to 4.5 when the pharmaceutical composition is dissolved in distilled water for injection.

As another aspect of the pharmaceutical composition of the formulation of the present invention, there is a pharmaceutical composition containing HMGB1 fragment peptide as an active ingredient or a trifluoroacetate salt thereof, citric acid, sodium citrate, and sucrose, and having a pH of 3.0 to 4.5 when the pharmaceutical composition is dissolved in distilled water for injection.

The formulation of the present invention can be produced by freeze-drying the preparation solution. Freeze-drying is a drying method in which an aqueous solution or dispersion containing an active ingredient is frozen, and water is sublimated and removed. That is, it is a method in which an aqueous solution or dispersion containing an active ingredient is frozen and dried by sublimation from the frozen surface under high vacuum.

The pharmaceutical composition of the formulation of the present invention is a freeze-dried pharmaceutical composition containing an HMGB1 fragment peptide as an active ingredient or an acid addition salt thereof, an organic acid, a metal salt of an organic acid, and a sugar and/or a sugar alcohol.

As another aspect of the pharmaceutical composition of the formulation of the present invention, there is a freeze-dried pharmaceutical composition containing an HMGB1 fragment peptide as an active ingredient or an acid addition salt thereof, an organic acid, a metal salt of an organic acid, and one or more selected from the group consisting of a monosaccharide, a disaccharide, and a polysaccharide as a sugar and/or a sugar alcohol.

As another aspect of the pharmaceutical composition of the formulation of the present invention, there is a freeze-dried pharmaceutical composition containing an HMGB1 fragment peptide as an active ingredient or an acid addition salt thereof, an organic acid, a metal salt of an organic acid, and one or more selected from the group consisting of glucose, fructose, sucrose, mannitol, and trehalose as a sugar and/or a sugar alcohol.

As another aspect of the pharmaceutical composition of the formulation of the present invention, there is a freeze-dried pharmaceutical composition containing an HMGB1 fragment peptide as an active ingredient or a trifluoroacetate salt thereof, an organic acid, a metal salt of an organic acid, and sucrose.

As another aspect of the pharmaceutical composition of the formulation of the present invention, there is a freeze-dried pharmaceutical composition containing an HMGB1 fragment peptide as an active ingredient or a trifluoroacetate salt thereof, citric acid, a metal salt of an organic acid, and sucrose.

As another aspect of the pharmaceutical composition of the formulation of the present invention, there is a freeze-dried pharmaceutical composition containing an HMGB1 fragment peptide as an active ingredient or a trifluoroacetate salt thereof, an organic acid, sodium citrate, and sucrose.

As another aspect of the pharmaceutical composition of the formulation of the present invention, there is a freeze-dried pharmaceutical composition containing HMGB1 fragment peptide as active ingredient or a trifluoroacetate salt thereof, citric acid, sodium citrate, and sucrose.

The pharmaceutical composition of the formulation of the present invention is a freeze-dried pharmaceutical composition containing an HMGB1 fragment peptide as an active ingredient or an acid addition salt thereof, an organic acid, a metal salt of an organic acid, and a sugar and/or a sugar alcohol, and is a pharmaceutical composition having a pH of 3.0 to 4.5 when the pharmaceutical composition is dissolved in distilled water for injection.

As another aspect of the pharmaceutical composition of the formulation of the present invention, there is a freeze-dried pharmaceutical composition containing an HMGB1 fragment peptide as an active ingredient or an acid addition salt thereof, an organic acid, a metal salt of an organic acid, and one or more selected from the group consisting of a monosaccharide, a disaccharide, and a polysaccharide as a sugar and/or a sugar alcohol, and there is a pharmaceutical composition having a pH of 3.0 to 4.5 when the pharmaceutical composition is dissolved in distilled water for injection.

As another aspect of the pharmaceutical composition of the formulation of the present invention, there is a freeze-dried pharmaceutical composition containing an HMGB1 fragment peptide as an active ingredient or an acid addition salt thereof, an organic acid, a metal salt of an organic acid, and one or more selected from the group consisting of glucose, fructose, sucrose, mannitol, and trehalose as a sugar and/or a sugar alcohol, and there is a pharmaceutical composition having a pH of 3.0 to 4.5 when the pharmaceutical composition is dissolved in distilled water for injection.

As another aspect of the pharmaceutical composition of the formulation of the present invention, there is a freeze-dried pharmaceutical composition containing HMGB1 fragment peptide as an active ingredient or a trifluoroacetate salt thereof, an organic acid, a metal salt of an organic acid, and sucrose, and there is a pharmaceutical composition having a pH of 3.0 to 4.5 when the pharmaceutical composition is dissolved in distilled water for injection.

As another aspect of the pharmaceutical composition of the formulation of the present invention, there is a freeze-dried pharmaceutical composition containing HMGB1 fragment peptide as an active ingredient or a trifluoroacetate salt thereof, citric acid, a metal salt of an organic acid, and sucrose, and there is a pharmaceutical composition having a pH of 3.0 to 4.5 when the pharmaceutical composition is dissolved in distilled water for injection.

As another aspect of the pharmaceutical composition of the formulation of the present invention, there is a freeze-dried pharmaceutical composition containing HMGB1 fragment peptide as an active ingredient or a trifluoroacetate salt thereof, an organic acid, sodium citrate, and sucrose, and there is a pharmaceutical composition having a pH of 3.0 to 4.5 when the pharmaceutical composition is dissolved in distilled water for injection.

As another aspect of the pharmaceutical composition of the formulation of the present invention, there is a freeze-dried pharmaceutical composition containing HMGB1 fragment peptide as an active ingredient or a trifluoroacetate salt thereof, citric acid, sodium citrate, and sucrose, and there is a pharmaceutical composition having a pH of 3.0 to 4.5 when the pharmaceutical composition is dissolved in distilled water for injection.

The pharmaceutical composition of the formulation of the present invention is a pharmaceutical composition produced by dissolving HMGB1 fragment peptide as an active ingredient or an acid addition salt thereof, a hydrate of an organic acid, a hydrate of a metal salt of an organic acid, and a sugar and/or a sugar alcohol in distilled water for injection, and adjusting the pH to 2.9 to 3.5 with a hydroxide.

The pharmaceutical composition of the formulation of the present invention is a pharmaceutical composition produced by dissolving HMGB1 fragment peptide as an active ingredient or an acid addition salt thereof, a hydrate of an organic acid, a hydrate of a metal salt of an organic acid, and one or more selected from the group consisting of a monosaccharide, a disaccharide and a polysaccharide as a sugar and/or a sugar alcohol in distilled water for injection, and adjusting the pH to 2.9 to 3.5 with a hydroxide.

Another aspect of the pharmaceutical composition of the formulation of the present invention is a pharmaceutical composition produced by dissolving HMGB1 fragment peptide as an active ingredient or an acid addition salt thereof, an organic acid, a metal salt of an organic acid, and one or more selected from the group consisting of glucose, fructose, sucrose, mannitol, and trehalose as a sugar and/or a sugar alcohol in distilled water for injection, and adjusting the pH to 2.9 to 3.5 with a hydroxide.

Another aspect of the pharmaceutical composition of the formulation of the present invention is a pharmaceutical composition produced by dissolving HMGB1 fragment peptide as an active ingredient or a trifluoroacetate salt thereof, an organic acid, a metal salt of an organic acid, and sucrose in distilled water for injection and adjusting the pH to 2.9 to 3.5 with a hydroxide.

Another aspect of the pharmaceutical composition of the formulation of the present invention is a pharmaceutical composition produced by dissolving HMGB1 fragment peptide as an active ingredient or a trifluoroacetate salt thereof, citric acid, a metal salt of an organic acid, and sucrose in distilled water for injection and adjusting the pH to 2.9 to 3.5 with a hydroxide.

Another aspect of the pharmaceutical composition of the formulation of the present invention is a pharmaceutical composition produced by dissolving HMGB1 fragment peptide as an active ingredient or a trifluoroacetate salt thereof, an organic acid, sodium citrate, and sucrose in distilled water for injection and adjusting the pH to 2.9 to 3.5 with a hydroxide.

Another aspect of the pharmaceutical composition of the formulation of the present invention is a pharmaceutical composition produced by dissolving HMGB1 fragment peptide as an active ingredient or a trifluoroacetate salt thereof, citric acid, sodium citrate, and sucrose in distilled water for injection and adjusting the pH to 2.9 to 3.5 with a hydroxide.

Another aspect of the pharmaceutical composition of the formulation of the present invention is a pharmaceutical composition produced by dissolving HMGB1 fragment peptide as an active ingredient or a trifluoroacetate salt thereof, citric acid, sodium citrate, and sucrose in distilled water for injection and adjusting the pH to 2.9 to 3.5 with sodium hydroxide.

The pharmaceutical composition of the formulation of the present invention is a pharmaceutical composition produced by dissolving HMGB1 fragment peptide as an active ingredient or an acid addition salt thereof, a hydrate of an organic acid, a hydrate of a metal salt of an organic acid and a sugar and/or a sugar alcohol in distilled water for injection, adjusting the pH to 2.9 to 3.5 with a hydroxide, and freeze-drying the solution.

The pharmaceutical composition of the formulation of the present invention is a pharmaceutical composition produced by dissolving HMGB1 fragment peptide as an active ingredient or an acid addition salt thereof, a hydrate of an organic acid, a hydrate of a metal salt of an organic acid, and one or more selected from the group consisting of a monosaccharide, a disaccharide and a polysaccharide as a sugar and/or a sugar alcohol in distilled water for injection, adjusting the pH to 2.9 to 3.5 with a hydroxide, and freeze-drying the solution.

Another aspect of the pharmaceutical composition of the formulation of the present invention is a pharmaceutical composition produced by dissolving HMGB1 fragment peptide as an active ingredient or an acid addition salt thereof, an organic acid, a metal salt of an organic acid, and one or more selected from the group consisting of glucose, fructose, sucrose (refined white sugar), mannitol, and trehalose as a sugar and/or a sugar alcohol in distilled water for injection, adjusting the pH to 2.9 to 3.5 with a hydroxide, and freeze-drying the solution.

Another aspect of the pharmaceutical composition of the formulation of the present invention is a pharmaceutical composition produced by dissolving HMGB1 fragment peptide as an active ingredient or a trifluoroacetate salt thereof, an organic acid, a metal salt of an organic acid, and sucrose in distilled water for injection, adjusting the pH to 2.9 to 3.5 with a hydroxide, and freeze-drying the solution.

Another aspect of the pharmaceutical composition of the formulation of the present invention is a pharmaceutical composition produced by dissolving HMGB1 fragment peptide as an active ingredient or a trifluoroacetate salt thereof, citric acid, a metal salt of an organic acid, and sucrose in distilled water for injection, adjusting the pH to 2.9 to 3.5 with a hydroxide, and freeze-drying the solution.

Another aspect of the pharmaceutical composition of the formulation of the present invention is a pharmaceutical composition produced by dissolving HMGB1 fragment peptide as an active ingredient or a trifluoroacetate salt thereof, an organic acid, sodium citrate, and sucrose in distilled water for injection, adjusting the pH to 2.9 to 3.5 with a hydroxide, and freeze-drying the solution.

Another aspect of the pharmaceutical composition of the formulation of the present invention is a pharmaceutical composition produced by dissolving HMGB1 fragment peptide as an active ingredient or a trifluoroacetate salt thereof, a citric acid, sodium citrate, and sucrose in distilled water for injection, adjusting the pH to 2.9 to 3.5 with a hydroxide, and freeze-drying the solution.

Another aspect of the pharmaceutical composition of the formulation of the present invention is a pharmaceutical composition produced by dissolving HMGB1 fragment peptide as an active ingredient or a trifluoroacetate salt thereof, citric acid, sodium citrate, and sucrose in distilled water for injection, adjusting the pH to 2.9 to 3.5 with sodium hydroxide, and freeze-drying the solution.

It is noted that the descriptions of all prior art references cited herein are incorporated herein by reference.

### [EXAMPLES]

The present invention will be explained in more detail below by way of Examples, Comparative Examples and Reference Examples, but these do not limit the present invention. The formulations of Examples, Comparative Examples and Reference Examples were produced by the following methods.

### (1) Study of pH of preparation solution

The formulation of the present invention is produced by freeze-drying the preparation solution. Therefore, the influence of the difference in pH of the preparation solution on the quality of the freeze-dried formulation was evaluated. The active ingredient (Hereinafter, it may be referred to as "drug substance".) refers to a substance described in any one of the following (a) to (c).
(a) an HMGB1 fragment peptide comprising the amino acid sequence set forth in SEQ ID NO: 1 or an acid addition salt thereof;
(b) a peptide comprising an amino acid sequence having substitution, deletion, insertion or addition of one or more amino acids in the amino acid sequence set forth in SEQ ID NO: 1, and having an activity of stimulating migration of a cell, or an acid addition salt thereof; and
(c) a peptide comprising an amino acid sequence having about 80% or more of sequence identity to the amino acid sequence set forth in SEQ ID NO: 1, and having an activity of stimulating migration of a cell, or an acid addition salt thereof.

### (Experimental method)

### a. Process for formulation production

Table 1 shows the prescription of the formulation. Citric acid hydrate (manufactured by Merck) and sodium citrate hydrate (manufactured by Merck) were each weighed in a predetermined amount and dissolved in water for injection. These solutions were mixed to adjust the pH to a predetermined value, thereby a citrate buffer solution with a predetermined concentration was prepared. A predetermined amount of the drug substance and polysorbate 80 (manufactured by Merck Corporation) were dissolved in the citrate buffer solution, then the final pH was adjusted with an aqueous sodium hydroxide solution, and the liquid was stirred, and the liquid amount was adjusted to the final weight with a citrate buffer solution.

The prepared liquid was filtered through a hydrophilic PVDF filter (manufactured by Merck Millipore), the filtrate was then filled into a 3 mL vial (manufactured by Taisei Kako Co., Ltd.), and the vial was half-sealed with a rubber stopper. Thereafter, freeze-drying was performed using a freeze-dryer under the following conditions. After completion of the freeze-drying, the inside of the freeze-dryer was decompressed by nitrogen, and the rubber stopper was fully sealed and capped.

### (Conditions for freeze-drying)

A freeze-dried product was produced by performing 1) cooling at 5°C, 2) cooling at -5°C for 1 hour, 3) freezing at -40°C for 4 hours, 4) primary drying at -35°C and a vacuum pressure of 10 Pa for 24 hours or more, and 6) secondary drying at 25°C and a vacuum pressure of 2 Pa for 5 hours or more.

**[Table 1]**

| | Example 1 | Example 2 | Comparative Example 1 | Comparative Example 2 |
|---|---|---|---|---|
| Component | Quantity (mg/g) | | | |
| Drug substance*¹ | 20.0 | 20.0 | 20.0 | 20.0 |
| Polysorbate 80 | 5.0 | 5.0 | 5.0 | 5.0 |
| Citrate buffer solution*² (Buffer solution pH) | Adequate amount (pH 3.0) | Adequate amount (pH 4.0) | Adequate amount (pH 4.5) | Adequate amount (pH 5.0) |
| Sodium hydroxide | Adequate amount | Adequate amount | Adequate amount | Adequate amount |
| Preparation solution pH | 3.0 | 4.0 | 4.5 | 5.0 |

| | | | | |
|---|---|---|---|---|
| *¹: As free form *²: A 20 mmol/L citric acid hydrate solution and a 20 mmol/L sodium citrate hydrate solution were mixed to have a predetermined pH. | | | | |

### b. Method for measuring related substance

An related substance was measured by UPLC (model: ACQUITY (manufactured by Waters Corporation)).

### (1) Preparation method of sample dilution solvent

Cetyltrimethylammonium chloride was dissolved in a proportion of 0.16 g to a preparation amount of 500 mL to prepare a 0.001 M sample dilution solvent.

### (2) Preparation method of standard solution

The drug substance in the same lot as the sample was dissolved in a sample dilution solvent to prepare a concentration of about 0.5 mg/mL.

### (3) Method for preparing sample solution of preparation solution

The preparation solution was dissolved in a sample dilution solvent so that the concentration of the drug substance was 0.5 mg/mL to prepare a sample solution.

### (4) Method for preparing sample solution of freeze-dried formulation

One vial of the freeze-dried formulation was dissolved in a sample dilution solvent so that the concentration of the drug substance was 0.5 mg/mL to prepare a sample solution. The amount of the related substance was calculated by an area percentage method.

### Test conditions

Detector: Ultraviolet absorptiometer (measurement wavelength: 220 nm)
Column: AQUITY UPLC BEH300 C18, 2.1 × 150 mm, 1.7 µm (Waters)
Column temperature: Constant temperature around 70°C
Mobile phase A: I: Water/Trifluoroacetic acid for liquid chromatography (1000 : 1)
II: Prepare acetonitrile for liquid chromatography/trifluoroacetic acid for liquid chromatography (1000 : 1), mix I/II (95 : 5)
Mobile phase B: I: Water/Trifluoroacetic acid for liquid chromatography (1000 : 1),
II: Prepare acetonitrile for liquid chromatography/trifluoroacetic acid for liquid chromatography (1000 : 1), mix I/II (75 : 25)

The gradient programs for mobile phases A and B are as shown in Table 2.

**[Table 2]**

| UPLC related substance gradient program | | |
|---|---|---|
| Time (min) from sample injection | Mobile phase A [%] | Mobile phase B [%] |
| 0 | 100 | 0 |
| 2 | 100 | 0 |
| 22 | 10 | 90 |
| 30 | 10 | 90 |
| 35 | 0 | 100 |
| 45 | 0 | 100 |
| 45.1 | 100 | 0 |
| 55 | 100 | 0 |

| | | |
|---|---|---|
| Flow rate: 0.2 mL/min Injected amount: 5 pL Sample cooler temperature: Constant temperature around 5°C Area measurement range: 55 minutes after sample injection | | |

### c. pH measurement method

While stirring the preparation solution, the pH was measured by a pH meter (manufactured by HORIBA, Ltd.).

### (Experimental results)

Table 3 shows the experimental results. As a result, in the freeze-dried formulation, the lower the pH of the preparation solution, the lower the dimer amount and the total related substance amount, and the quality was improved. In particular, it was found that the amount of the dimer or the total related substance can be reduced in the vicinity of a pH of 3 to 4.

**[Table 3]**

| | Example 1 | Example 2 | Comparative Example 1 | Comparative Example 2 |
|---|---|---|---|---|
| Dimer (%) | 0.64 | 2.44 | 4.32 | 5.70 |
| Total related substance (%) | 4.33 | 6.68 | 9.05 | 10.64 |

### (2) Study of stabilizer

The stabilizer of the formulation of the present invention was studied. As a stabilizer, refined white sugar of a non-reducing disaccharide was selected, and the relationship between the addition amount and the solid stability was evaluated.

### (Experimental method)

### a. Process for formulation production

Tables 4 and 5 show the prescription of the formulation. Citric acid hydrate and sodium citrate hydrate were weighed in predetermined amounts and dissolved in distilled water for injection. The pH of the solution was adjusted to 4.0 to prepare a citrate buffer solution having a predetermined concentration. The additive and the drug substance weighed were added to and dissolved in the citrate buffer solution, and the final pH was adjusted to 4.0 with an aqueous sodium hydroxide solution and stirred. The liquid amount was then adjusted to the final weight with a citrate buffer solution.

The prepared liquid was filtered through a hydrophilic PVDF filter (manufactured by Merck Millipore), the filtrate was then filled into a 3 mL vial (manufactured by Taisei Kako Co., Ltd.), and the vial was half-sealed with a rubber stopper. Thereafter, freeze-drying was performed using a freeze-dryer under the following conditions. After completion of the freeze-drying, the inside of the freeze-dryer was decompressed by nitrogen, and the rubber stopper was fully sealed and capped.

### (Conditions for freeze-drying)

A freeze-dried product was produced by performing 1) cooling at 5°C, 2) cooling at -5°C for 1 hour, 3) freezing at -40°C for 4 hours, 4) primary drying at -35°C and a vacuum pressure of 10 Pa for 24 hours or more, and 6) secondary drying at 25°C and a vacuum pressure of 2 Pa for 5 hours or more.

**[Table 4]**

| | Example 3 | Example 4 | Example 5 |
|---|---|---|---|
| Component | Quantity (mg/g) | | |
| Drug substance*¹ | 20.0 | 20.0 | 20.0 |
| Sucrose (refined white sugar) (Amount relative to drug substance free form) | 33.7 (25 times by mol) | 67.5 (50 times by mol) | 101.2 (75 times by mol) |
| Polysorbate 80 | 5.0 | 5.0 | 5.0 |
| Citrate buffer solution*² | Adequate amount | Adequate amount | Adequate amount |
| Sodium hydroxide | Adequate amount | Adequate amount | Adequate amount |
| Preparation solution pH | 4.0 | 4.0 | 4.0 |
| Solute concentration in solution (%)*3 | 6.6 | 10.0 | 13.3 |

| | | | |
|---|---|---|---|
| * 1: As free form *2: A 20 mmol/L citric acid hydrate solution and a 20 mmol/L sodium citrate hydrate solution were mixed to have a pH of 4.0. *3: The solute concentration was calculated from the added refined white sugar and 20 mmol/L citrate buffer solution (anhydrous citric acid molecular weight: 192.1235) in consideration of a conversion factor of 1.41 of the drug substance. | | | |

**[Table 5]**

| | Example 6 | Comparative Example 3 |
|---|---|---|
| Component | Quantity (mg/g) | |
| Drug substance*¹ | 20.0 | 20.0 |
| Sucrose (refined white sugar) (Amount relative to drug substance free form) | 134.9 (100 times by mol) | - |
| Polysorbate 80 | 5.0 | 5.0 |
| Citrate buffer solution*² | Adequate amount | Adequate amount |
| Sodium hydroxide | Adequate amount | Adequate amount |
| Preparation solution pH | 4.0 | 4.0 |
| Solute concentration in solution (%)*³ | 16.7 | 3.2 |

| | | |
|---|---|---|
| * 1: As free form *2: A 20 mmol/L citric acid hydrate solution and a 20 mmol/L sodium citrate hydrate solution were mixed to have a pH of 4.0. *3: The solute concentration was calculated from the added refined white sugar and 20 mmol/L citrate buffer solution (anhydrous citric acid molecular weight: 192.1235) in consideration of a conversion factor of 1.41 of the drug substance. | | |

### b. Stability over time

The freeze-dried formulation was stored in a thermo-hygrostat under 40°C/75% RH (relative humidity) for 1 month, and the amounts of a dimer and total related substance were measured initially and after storage over time.

### c. Method for measuring related substance

As in Examples 1 and 2, measurement was performed by UPLC.

### d. pH measurement method

While stirring the preparation solution, the pH was measured by a pH meter (manufactured by HORIBA, Ltd.).

### (Experimental results)

Table 6 to 8 shows the experimental results. As a result, as compared with Comparative Example 3 without a stabilizer, the formulations of Examples 3 to 6 containing refined white sugar improved the stability.

**[Table 6]**

| | Example 3 | | Example 4 | |
|---|---|---|---|---|
| Storage conditions | Dimer (%) | Total related substance (%) | Dimer (%) | Total related substance (%) |
| Initial | 3.19 | 6.96 | 2.60 | 6.74 |
| 40°C/75%RH/ 1 month later (after storage over time) | 3.84 | 9.33 | 3.11 | 8.10 |
| Increased amount from initial | 0.65 | 2.37 | 0.51 | 1.36 |

**[Table 7]**

| | Example 5 | | Example 6 | |
|---|---|---|---|---|
| Storage conditions | Dimer (%) | Total related substance (%) | Dimer (%) | Total related substance (%) |
| Initial | 2.22 | 5.55 | 2.35 | 5.90 |
| 40°C/75%RH/ 1 month later (after storage over time) | 2.75 | 7.56 | 2.84 | 7.42 |
| Increased amount from initial | 0.53 | 2.01 | 0.49 | 1.52 |

**[Table 8]**

| | Comparative Example 3 | |
|---|---|---|
| Storage conditions | Dimer (%) | Total related substance (%) |
| Initial | 2.17 | 5.85 |
| 40°C/75%RH/ 1 month | 3.78 | 9.77 |
| Increased amount from initial | 1.61 | 3.92 |

### (3) Study of solution stability at pH of preparation solution

It became clear that the difference in pH of the preparation solution affects the stability of the active ingredient, and in particular, it became clear that the amount of the related substance can be reduced at a pH of 3 to 4. Therefore, the stability was actually studied with the preparation solution having a pH around the pH.

### (Experimental method)

### a. Process for formulation production

Tables 9 and 10 show the prescription of the formulation. Water for injection was weighed into a beaker and then additive was added and dissolved. After cooling the solution to 5°C with a cool stirrer, a predetermined amount of the active ingredient was added and the pH of the solution was adjusted to 2.7, 3.2, 3.5, 3.7, 4.0 with 0.5 N sodium hydroxide. After adjusting the pH, the final weight was adjusted with water for injection. The refined white sugar was blended at a 25 times by mol the original drug substance. The preparation solution was stored at 25°C for 24 hours.

**[Table 9]**

| | Example 7 | Example 8 | Example 9 |
|---|---|---|---|
| Component | Quantity (mg/g) | | |
| Drug substance*¹ | 20.0 | 20.0 | 20.0 |
| Citric acid hydrate | 3.9 | 3.4 | 3.1 |
| Sodium citrate hydrate | 0.4 | 1.1 | 1.6 |
| Sucrose (refined white sugar) | 33.7 | 33.7 | 33.7 |
| Polysorbate 80 | 5.0 | 5.0 | 5.0 |
| Sodium hydroxide | Adequate amount | Adequate amount | Adequate amount |
| Preparation solution pH | 2.7 | 3.2 | 3.5 |

| | | | |
|---|---|---|---|
| *¹: As free form | | | |

**[Table 10]**

| | Example 10 | Reference Example 1 |
|---|---|---|
| Component | Quantity (mg/g) | |
| Drug substance*¹ | 20.0 | 20.0 |
| Citric acid hydrate | 2.9 | 2.6 |
| Sodium citrate hydrate | 1.8 | 2.2 |
| Sucrose (refined white sugar) | 33.7 | 33.7 |
| Polysorbate 80 | 5.0 | 5.0 |
| Sodium hydroxide | Adequate amount | Adequate amount |
| Preparation solution pH | 3.7 | 4.0 |

| | | |
|---|---|---|
| ^{*1}: As free form | | |

### b. Method for measuring related substance

As in Examples 1 and 2, measurement was performed by UPLC.

### c. pH measurement method

While stirring the preparation solution, the pH was measured by a pH meter (manufactured by HORIBA, Ltd.).

### (Experimental results)

Table 11 to 13 shows the experimental results. As a result, as the pH of the solution was increased, both the dimer and the total related substance were significantly increased. In particular, the preparation solution having a pH of 4.0 had the worst stability.

**[Table 11]**

| | Example 7 | | Example 8 | |
|---|---|---|---|---|
| Elapsed time (Time) | Dimer (%) | Total related substance (%) | Dimer (%) | Total related substance (%) |
| 0 | 0.48 | 4.07 | 0.51 | 4.06 |
| 24 | 0.79 | 4.84 | 1.36 | 6.13 |
| Increased amount from 0 hour | 0.31 | 0.77 | 0.85 | 2.07 |

**[Table 12]**

| | Example 9 | | Example 10 | |
|---|---|---|---|---|
| Elapsed time (Time) | Dimer (%) | Total related substance (%) | Dimer (%) | Total related substance (%) |
| 0 | 0.67 | 4.03 | 0.51 | 4.42 |
| 24 | 1.87 | 7.39 | 3.77 | 11.64 |
| Increased amount from 0 hour | 1.20 | 3.36 | 3.26 | 7.22 |

**[Table 13]**

| | Reference Example 1 | |
|---|---|---|
| Elapsed time (Time) | Dimer (%) | Total related substance (%) |
| 0 | 0.62 | 4.43 |
| 24 | 7.72 | 18.95 |
| Increased amount from 0 hour | 7.10 | 14.52 |

### (4) Study on influence of additive

The influence on the stability of the freeze-dried formulation was evaluated by the type and amount of the additive in the formulation of the present invention.

### (Experimental method)

### a. Process for formulation production

Tables 14 and 15 show the prescription of the formulation. Water for injection was weighed into a beaker and then additive was added and dissolved. This solution was cooled to 5°C with a cool stirrer, then a predetermined amount of the active ingredient was added, and the pH was measured. Thereafter, the pH was adjusted to 2.7 with a 0.5 N sodium hydroxide solution. After adjusting the pH, the final weight was adjusted with water for injection.

The prepared liquid was filtered through a hydrophilic PVDF filter (manufactured by Merck Millipore), the filtrate was then filled into a 3 mL vial (manufactured by Taisei Kako Co., Ltd.), and the vial was half-sealed with a rubber stopper. Thereafter, freeze-drying was performed under the following conditions. After completion of freeze-drying, the inside of the freeze-dryer chamber was decompressed by nitrogen, and the rubber stopper was fully sealed and capped.

### (Conditions for freeze-drying)

A freeze-dried product was produced by performing 1) cooling at 5°C, 2) cooling at -5°C for 1 hour, 3) freezing at -40°C for 4 hours, 4) primary drying at -35°C and a vacuum pressure of 10 Pa for 24 hours or more, and 6) secondary drying at 40°C and a vacuum pressure of 2 Pa for 5 hours or more.

**[Table 14]**

| | Example 11 | Example 12 | Example 13 |
|---|---|---|---|
| Component | Quantity (mg/vial) | | |
| Drug substance*¹ | 5.0 | 5.0 | 5.0 |
| Citric acid hydrate | 1.0 | 1.0 | 1.0 |
| Sodium citrate hydrate | 0.1 | 0.1 | 0.1 |
| Sucrose (refined white sugar) (Amount relative to drug substance free form) | 8.4 (25 times by mol) | 25.3 (75 times by mol) | 8.4 (25 times by mol) |
| Trehalose | - | - | - |
| Polysorbate 80 | 1.3 | 1.3 | - |
| Sodium hydroxide | Adequate amount | Adequate amount | Adequate amount |
| Preparation solution pH | 2.7 | 2.7 | 2.7 |

| | | | |
|---|---|---|---|
| ^{*1}: As free form | | | |

**[Table 15]**

| | Example 14 | Comparative Example 4 | Comparative Example 5 |
|---|---|---|---|
| Component | Quantity (mg/vial) | | |
| Drug substance*¹ | 5.0 | 5.0 | 5.0 |
| Citric acid hydrate | 1.0 | 1.0 | 1.0 |
| Sodium citrate hydrate | 0.1 | 0.1 | 0.1 |
| Sucrose (refined white sugar) (Amount relative to drug substance free form) | 25.3 (75 times by mol) | - | - |
| Trehalose | - | 2.9 | 20.9 |
| Polysorbate 80 | - | 1.3 | 1.3 |
| Sodium hydroxide | Adequate amount | Adequate amount | Adequate amount |
| Preparation solution pH | 2.7 | 2.7 | 2.7 |

| | | | |
|---|---|---|---|
| *¹: As free form | | | |

### b. Stability over time

The freeze-dried formulation was stored in a thermo-hygrostat under 40°C/75% RH (relative humidity) for 2 weeks, and the amounts of a dimer and total related substance were measured.

### c. Method for measuring related substance

As in Examples 1 and 2, measurement was performed by UPLC.

### d. pH measurement method

While stirring the preparation solution, the pH was measured by a pH meter (manufactured by HORIBA, Ltd.).

### (Experimental results)

Table 16 to 18 shows the experimental results. As a result, as compared with the formulation of Examples 11 to 14 containing refined white sugar, the formulations containing trehalose of Comparative Examples 4 and 5 increased the dimer amount and the total related substance amount after storage over time. On the other hand, the dimer amounts and total related substance amounts of Examples 11 and 12 containing polysorbate 80 and refined white sugar as stabilizers and Examples 13 and 14 without polysorbate 80 and containing refined white sugar were almost the same. Therefore, it has become clear that the related substance amount can be sufficiently reduced even with only refined white sugar as the stabilizer.

**[Table 16]**

| | Example 11 | | Example 12 | |
|---|---|---|---|---|
| | Dimer (%) | Total related substance (%) | Dimer (%) | Total related substance (%) |
| Initial | 0.52 | 3.70 | 0.69 | 3.03 |
| 40°C/75%RH/ 2 weeks later (after storage over time) | 1.02 | 5.15 | 0.85 | 3.94 |
| Increased amount from initial | 0.50 | 1.45 | 0.16 | 0.91 |

**[Table 17]**

| | Example 13 | | Example 14 | |
|---|---|---|---|---|
| | Dimer (%) | Total related substance (%) | Dimer (%) | Total related substance (%) |
| Initial | 0.58 | 3.99 | 0.59 | 3.15 |
| 40°C/75%RH/ 2 weeks later (after storage over time) | 0.90 | 4.01 | 0.75 | 3.33 |
| Increased amount from initial | 0.32 | 0.92 | 0.16 | 0.18 |

**[Table 18]**

| | Comparative Example 4 | | Comparative Example 5 | |
|---|---|---|---|---|
| | Dimer (%) | Total related substance (%) | Dimer (%) | Total related substance (%) |
| Initial | 0.63 | 3.74 | 0.57 | 3.37 |
| 40°C/75%RH/ 2 weeks later (after storage over time) | 3.30 | 8.53 | 1.24 | 4.86 |
| Increased amount from initial | 2.67 | 4.79 | 0.67 | 1.49 |

### (5) Study of concentration of preparation solution

The influence on the stability of the freeze-dried formulation was evaluated by the concentration of the preparation solution in the formulation of the present invention.

### (Experimental method)

### a. Process for formulation production

Table 19 shows the prescription of the formulation. Water for injection was weighed in a beaker, and then citric acid hydrate, sodium citrate hydrate and refined white sugar were added and dissolved. The pH of this solution was measured, then the drug substance was added and dissolved, and the pH was measured again. Thereafter, a sodium hydroxide solution was added so as to have a pH of 3.2, the pH was adjusted, and then the final weight was adjusted with water for injection.

The prepared liquid was filtered through a hydrophilic PVDF filter (manufactured by Merck Millipore), the filtrate was then filled in a vial (manufactured by FUJI GLASS CO.,LTD.), and the vial was half-sealed with a rubber stopper. Thereafter, freeze-drying was performed using a freeze-dryer under the following conditions. After completion of the freeze-drying, the inside of the freeze-dryer was decompressed by nitrogen, and the rubber stopper was fully sealed and capped.

**[Table 19]**

| | Example 15 | Example 16 |
|---|---|---|
| Component | Quantity (mg/vial) | |
| Drug substance*¹ | 15.0 | 15.0 |
| Citric acid hydrate | 2.6 | 2.6 |
| Sodium citrate hydrate | 0.8 | 0.8 |
| Sucrose (refined white sugar) (Amount relative to drug substance free form) | 75.9 (75 times by mol) | 75.9 (75 times by mol) |
| Sodium hydroxide | Adequate amount | Adequate amount |
| Preparation solution pH | 3.2 | 3.2 |
| Final concentration of preparation solution | 20mg/g | 10mg/g |
| Filling amount | 0.75g | 1.50g |

| | | |
|---|---|---|
| ^{*1} As free form | | |

### (Conditions for freeze-drying)

As shown in Tables 20 and 21, 1) cooling at 5°C, 2) cooling at -5°C for 2 hours, 3) freezing at -40°C for 4 hours, 4) primary drying at -33°C for 45 hours or more at a vacuum pressure of 10 Pa, and 6) secondary drying at 40°C for 8 hours or more at a vacuum pressure of 5 Pa were performed to produce a freeze-dried product.

**[Table 20]**

| Preliminary freezing 1 | | Preliminary freezing 2 | | Freezing | | |
|---|---|---|---|---|---|---|
| Temperature (°C) | Time (min) | Temperature (°C) | Time (min) | Cooling rate (°C/min) | Temperature (°C) | Time (min) |
| 5 | 60 | -5 | 120 | 1.0 | -40 | 240 |

**[Table 21]**

| Primary drying | | | Secondary drying | | |
|---|---|---|---|---|---|
| Temperature (°C) | Pressure (Pa) | Time (min) | Temperature (°C) | Pressure (Pa) | Time (min) |
| -33 | 10 | 4260 | 40 | 5 | 480 |

### b. Stability over time

The freeze-dried formulation was stored in a thermo-hygrostat under 25°C/60% RH (relative humidity) for 26 weeks, and the dimer amount and the total related substance amount were measured.

### c. Method for measuring related substance

An related substance was measured by HPLC (model: Alliance (manufactured by Waters Corporation)).

### (Preparation method of sample dilution solvent)

Cetyltrimethylammonium chloride was dissolved in a proportion of 0.16 g to a preparation amount of 500 mL to prepare a 0.001 M sample dilution solvent.

### (Sample solution preparation method)

To one vial, 3 mL of the sample dilution solvent was added to be redissolved, 1.5 mL of the solution was taken, and 3.5 mL of the sample dilution solvent was added to obtain a sample solution. The amount of the related substance was calculated by an area percentage method.

### Test conditions

Detector: Ultraviolet absorptiometer (measurement wavelength: 215 nm)
Column: XBridge Peptide BEH C18, 300Å, 4.6 × 250 mm, 5 pm (Waters)
Column temperature: Constant temperature around 55°C
Mobile phase A: Trifluoroacetic acid test solution
Mobile phase B: Acetonitrile for liquid chromatography/2-propanol for liquid chromatography/trifluoroacetic acid mixed solution (500: 500: 1)

The gradient programs for mobile phases A and B are as shown in Table 22.

**[Table 22]**

| HPLC related substance gradient program | | |
|---|---|---|
| Time (min) from sample injection | Mobile phase A (%) | Mobile phase B (%) |
| 0∼5 | 90 | 10 |
| 5∼65 | 90 → 75 | 10 → 25 |
| 65∼70 | 75 | 25 |
| 70∼70.1 | 75 → 90 | 25 → 10 |
| 70.1∼85 (Equilibration) | 90 | 10 |

| | | |
|---|---|---|
| Flow rate: 1.0 mL/min Injected amount: 20 pL Sample cooler temperature: Constant temperature around 5°C Area measurement range: 70 minutes after sample injection | | |

### d. pH measurement method

While stirring the preparation solution, the pH was measured by a pH meter (manufactured by HORIBA, Ltd.).

### (Experimental results)

Table 23 shows the experimental results. As a result, it has become clear that the concentration of the preparation solution does not affect the stability even when the final concentration of the preparation solution is changed, the freeze-dried formulation is produced using the preparation solution, and the freeze-dried formulation is stored over time.

**[Table 23]**

| | Example 15 | | Example 16 | |
|---|---|---|---|---|
| | Dimer (%) | Total related substance (%) | Dimer (%) | Total related substance (%) |
| Initial | 0.12 | 1.06 | 0.08 | 1.09 |
| 25°C/60%RH/ 26 weeks later (after storage over time) | 0.09 | 1.72 | 0.06 | 1.75 |

### (6) Study on increase in concentration of preparation solution and formulation with increased amount of sucrose

In the formulation of the present invention, a formulation in which the concentration of the preparation solution was increased and the amount of sucrose was increased was studied.

### (Experimental method)

### a. Process for formulation production

Table 24 to 26 show the prescription of the formulation. Water for injection was weighed in a beaker, and then citric acid hydrate, sodium citrate hydrate and refined white sugar were added and dissolved. The pH of this solution was measured, then the drug substance was added and dissolved, and the pH was measured again. Thereafter, the final weight was adjusted with water for injection and stirred.

The prepared liquid was filtered through a hydrophilic PVDF filter (manufactured by Merck Millipore), and then 0.25 g of the filtrate was filled in each vial (manufactured by Taisei Kako Co., Ltd.), and the vial was half-sealed with a rubber stopper. Thereafter, freeze-drying was performed using a freeze-dryer under the following conditions. After completion of the freeze-drying, the inside of the freeze-dryer was decompressed by nitrogen, and the rubber stopper was fully sealed and capped.

**[Table 24]**

| | Example 17 | Example 18 | Example 19 |
|---|---|---|---|
| Component | Quantity (mg/g) | | |
| Drug substance*¹ | 10.0 | 20.0 | 20.0 |
| Citric acid hydrate | 1.34 | 2.67 | 2.67 |
| Sodium citrate hydrate | 1.07 | 2.14 | 2.14 |
| Sucrose (refined white sugar) (Amount relative to drug substance free form) | 50.6 (75 times by mol) | 303.6 (225 times by mol) | 404.8 (300 times by mol) |
| Water for injection | Adequate amount | Adequate amount | Adequate amount |
| Preparation solution pH | 3.2 | 3.2 | 3.2 |

| | | | |
|---|---|---|---|
| *1 As free form | | | |

**[Table 25]**

| | Example 20 | Example 21 | Example 22 |
|---|---|---|---|
| Component | Quantity (mg/g) | | |
| Drug substance*¹ | 30.0 | 30.0 | 40.0 |
| Citric acid hydrate | 4.01 | 4.01 | 5.34 |
| Sodium citrate hydrate | 3.21 | 3.21 | 4.29 |
| Sucrose (refined white sugar) (Amount relative to drug substance free form) | 303.6 (150 times by mol) | 404.8 (200 times by mol) | 202.4 (75 times by mol) |
| Water for injection | Adequate amount | Adequate amount | Adequate amount |
| Preparation solution pH | 3.2 | 3.2 | 3.2 |

| | | | |
|---|---|---|---|
| *1 As free form | | | |

**[Table 26]**

| | Example 23 | Example 24 |
|---|---|---|
| Component | Quantity (mg/g) | |
| Drug substance*¹ | 40.0 | 40.0 |
| Citric acid hydrate | 5.34 | 5.34 |
| Sodium citrate hydrate | 4.29 | 4.29 |
| Sucrose (refined white sugar) (Amount relative to drug substance free form) | 303.6 (113 times by mol) | 404.8 (150 times by mol) |
| Water for injection | Adequate amount | Adequate amount |
| Preparation solution pH | 3.2 | 3.2 |

| | | |
|---|---|---|
| * 1 As free form | | |

### (Conditions for freeze-drying)

In Examples 17, 18, 22, and 23 freeze-drying was performed as shown in Tables 27 and 28, and in Examples 19, 20, 21, and 24 freeze-drying was performed as shown in Tables 29 and 30 to produce freeze-dried products.

**[Table 27]**

| Preliminary freezing 1 | | Preliminary freezing 2 | | Freezing | |
|---|---|---|---|---|---|
| Temperature (°C) | Time (min) | Temperature (°C) | Time (min) | Temperature (°C) | Time (min) |
| 5 | 60 | -5 | 120 | -40 | 240 |

**[Table 28]**

| Primary drying | | | Secondary drying | | |
|---|---|---|---|---|---|
| Temperature (°C) | Pressure (Pa) | Time (min) | Temperature (°C) | Pressure (Pa) | Time (min) |
| -30 | 10 | 5620 | 40 | 10 | 480 |

**[Table 29]**

| Preliminary freezing 1 | | Preliminary freezing 2 | | Freezing | |
|---|---|---|---|---|---|
| Temperature (°C) | Time (min) | Temperature (°C) | Time (min) | Temperature (°C) | Time (min) |
| 5 | 60 | -5 | 120 | -40 | 240 |

**[Table 30]**

| Primary drying | | | Secondary drying | | |
|---|---|---|---|---|---|
| Temperature (°C) | Pressure (Pa) | Time (min) | Temperature (°C) | Pressure (Pa) | Time (min) |
| -35 | 10 | 5664 | 40 | 10 | 480 |

### b. Stability over time

The freeze-dried formulation was stored in a thermo-hygrostat under 40°C/75% RH (relative humidity) for 1 month, and the dimer amount and the total related substance amount were measured.

### c. Method for measuring related substance

As in Examples 15 and 16, measurement was performed by HPLC.

### (Experimental results)

The amount of the dimer (initial, 40°C/75% RH (relative humidity)/increased amount from initial after storage for 1 month (after storage over time)) is shown in Tables 31 and 32, and the total related substance amount (initial, 40°C/75% RH (relative humidity)/increased amount from initial after storage for 1 month (after storage over time)) is shown in Tables 33 and 34. As a result, there was almost no difference in the increased amount of the dimer from the initial among the formulations, but the total related substance amount tended to decrease as the amount of sucrose increased.

**[Table 31]**

| Dimer amount (%) | Example 17 | Example 18 | Example 19 | Example 20 |
|---|---|---|---|---|
| Initial | 0.03 | 0.08 | 0.12 | 0.14 |
| 40°C/75%RH/ 1 month later (after storage over time) | 0.04 | 0.10 | 0.13 | 0.15 |
| Increased amount from initial | 0.02 | 0.01 | 0.01 | 0.01 |

**[Table 32]**

| Dimer amount (%) | Example 21 | Example 22 | Example 23 | Example 24 |
|---|---|---|---|---|
| Initial | 0.15 | 0.12 | 0.13 | 0.21 |
| 40°C/75%RH/ 1 month later (after storage over time) | 0.15 | 0.11 | 0.12 | 0.21 |
| Increased amount from initial | 0.00 | 0.00 | 0.00 | 0.00 |

**[Table 33]**

| Total related substance amount (%) | Example 17 | Example 18 | Example 19 | Example 20 |
|---|---|---|---|---|
| Initial | 1.30 | 1.36 | 1.41 | 1.50 |
| 40°C/75%RH/ 1 month later (after storage over time) | 1.82 | 1.75 | 1.91 | 2.09 |
| Increased amount from initial | 0.52 | 0.39 | 0.50 | 0.58 |

**[Table 34]**

| Total related substance amount (%) | Example 21 | Example 22 | Example 23 | Example 24 |
|---|---|---|---|---|
| Initial | 1.45 | 1.33 | 1.38 | 1.49 |
| 40°C/75%RH/ 1 month later (after storage over time) | 1.93 | 2.02 | 1.90 | 2.03 |
| Increased amount from initial | 0.48 | 0.69 | 0.52 | 0.55 |

### (7) Study of high-dose formulation with increased concentration of preparation solution

Quality evaluation was performed with a high-dose formulation (120 mg formulation) that is expected to be used in clinical trials.

### (Experimental method)

### a. Process for formulation production

Table 35 shows the prescription of the preparation solution, and Table 36 shows the prescription of 120 mg of the active ingredient per vial. Water for injection was weighed in a beaker, and then citric acid hydrate, sodium citrate hydrate and refined white sugar were added and dissolved. The pH of this solution was measured, then the drug substance was added and dissolved, and the pH was measured again. Thereafter, a sodium hydroxide solution was added so as to have a pH of 3.2, the pH was adjusted, and then the final weight was adjusted with water for injection. The prepared liquid was filtered through a hydrophilic PVDF filter (manufactured by Merck Millipore), the filtrate was then filled in a vial (manufactured by FUJI GLASS CO.,LTD.), and the vial was half-sealed with a rubber stopper. Thereafter, freeze-drying was performed using a freeze-dryer under the conditions of Tables 37 and 38. After completion of the freeze-drying, the inside of the freeze-dryer was decompressed by nitrogen, and the rubber stopper was fully sealed and capped. The filling amount of one vial was 3.0 g in Example 25, 4.0 g in Example 26, and 0.25 g in Example 22 as a control.

**[Table 35]**

| | Example 25 | Example 26 | Example 22 |
|---|---|---|---|
| Component | Quantity (mg/g) | | |
| Drug substance*¹ | 40.0 | 30.0 | 40.0 |
| Citric acid hydrate | 6.13 | 4.60 | 5.34 |
| Sodium citrate hydrate | 3.12 | 2.40 | 4.29 |
| Sucrose (refined white sugar) (Amount relative to drug substance free form) | 202.4 (75 times by mol) | 151.8 (75 times by mol) | 202.4 (75 times by mol) |
| Sodium hydroxide | Adequate amount | Adequate amount | Adequate amount |
| Water for injection | Adequate amount | Adequate amount | Adequate amount |
| Preparation solution pH | 3.2 | 3.2 | 3.2 |

| | | | |
|---|---|---|---|
| * 1 As free form | | | |

**[Table 36]**

| | Example 25 | Example 26 | Example 22 |
|---|---|---|---|
| Component | Quantity (mg/vial) | | |
| Drug substance^{*1} | 120.0 | 120.0 | 10.0 |
| Citric acid hydrate | 18.39 | 18.40 | 1.335 |
| Sodium citrate hydrate | 9.63 | 9.60 | 1.073 |
| Sucrose (refined white sugar) | 607.2 | 607.2 | 50.60 |
| Filling amount (g) | 3.00 | 4.00 | 0.25 |

| | | | |
|---|---|---|---|
| * 1 As free form | | | |

**[Table 37]**

| Preliminary freezing 1 | | Preliminary freezing 2 | | Freezing | | |
|---|---|---|---|---|---|---|
| Temperature (°C) | Time (min) | Temperature (°C) | Time (min) | Cooling rate (°C/min) | Temperature (°C) | Time (min) |
| 5 | 60 | -5 | 120 | 0.5 | -40 | 240 |

**[Table 38]**

| Primary drying | | | Secondary drying | | |
|---|---|---|---|---|---|
| Temperature (°C) | Pressure (Pa) | Time (min) | Temperature (°C) | Pressure (Pa) | Time (min) |
| -30 | 10 | 9534 | 40 | 10 | 480 |

### b. Stability over time

The freeze-dried formulation was stored in a thermo-hygrostat under 40°C/75% RH (relative humidity) for 1 month, and the dimer amount and the total related substance amount were measured.

### c. Method for measuring related substance

As in Examples 15 and 16, measurement was performed by HPLC.

### (Experimental results)

The amount of the dimer (initial, 40°C/75% RH (relative humidity)/increased amount from initial after storage for 1 month (after storage over time)) is shown in Table 39, and the total related substance amount (initial, 40°C/75% RH (relative humidity)/increased amount from initial after storage for 1 month (after storage over time)) is shown in Table 40. As a result, there was almost no difference in the increased amount of the dimer from the initial and the increased amount of the total related substance amount among the formulations.

**[Table 39]**

| Dimer amount (%) | Example 25 | Example 26 | Example 22 |
|---|---|---|---|
| Initial | 0.18 | 0.15 | 0.12 |
| 40°C/75%RH/ 1 month later (after storage over time) | 0.19 | 0.16 | 0.11 |
| Increased amount from initial | 0.01 | 0.01 | 0.00 |

**[Table 40]**

| Total related substance amount (%) | Example 25 | Example 26 | Example 22 |
|---|---|---|---|
| Initial | 1.26 | 1.18 | 1.33 |
| 40°C/75%RH/ 1 month later (after storage over time) | 2.12 | 2.14 | 2.02 |
| Increased amount from initial | 0.86 | 0.96 | 0.69 |

### [INDUSTRIAL APPLICABILITY]

The formulation of the present invention containing the present drug substance as a peptide contained a stabilizer, particularly a saccharide, and could improve stability by adjusting the pH to a specific pH range. Thus, even an unstable peptide can be stabilized over time.

## Claims

1. A pharmaceutical composition comprising the substance according to any one of the following (a) to (c), wherein a pH is 3.0 to 4.5 when the pharmaceutical composition is dissolved in distilled water for injection,
(a) an HMGB1 fragment peptide comprising the amino acid sequence set forth in SEQ ID NO: 1 or an acid addition salt thereof;
(b) a peptide comprising an amino acid sequence having substitution, deletion, insertion or addition of one or more amino acids in the amino acid sequence set forth in SEQ ID NO: 1, and having an activity of stimulating migration of a cell, or an acid addition salt thereof; and
(c) a peptide comprising an amino acid sequence having about 80% or more of sequence identity to the amino acid sequence set forth in SEQ ID NO: 1, and having an activity of stimulating migration of a cell, or an acid addition salt thereof.

2. The pharmaceutical composition according to claim 1, comprising one or more selected from the group consisting of an organic acid, an alkali metal salt of an organic acid, and an alkaline earth metal salt of an organic acid.

3. The pharmaceutical composition according to claim 2, comprising an organic acid, wherein the organic acid is one or more selected from the group consisting of citric acid, acetic acid, and phosphoric acid.

4. The pharmaceutical composition according to claim 2, comprising an organic acid and/or an alkali metal salt of the organic acid, wherein the organic acid and the alkali metal salt of the organic acid are citric acid and sodium citrate, respectively.

5. The pharmaceutical composition of any of claim 1 to 4, comprising a sugar and/or a sugar alcohol.

6. The pharmaceutical composition according to claim 5, comprising a sugar, wherein the sugar is one or more selected from the group consisting of a monosaccharide, a disaccharide, and a polysaccharide.

7. The pharmaceutical composition according to claim 5, wherein the sugar and/or sugar alcohol is one or more selected from the group consisting of glucose, fructose, sucrose, mannitol, and trehalose.

8. The pharmaceutical composition according to claim 7, wherein the sugar and/or sugar alcohol is sucrose.

9. The pharmaceutical composition according to any of claims 5 to 8, wherein the amount of sugar and/or sugar alcohol in the pharmaceutical composition is 10 to 300 times the amount of the substance according to any of (a) to (c) by mole.

10. The pharmaceutical composition according to claim 9, wherein the amount of the sugar and/or sugar alcohol in the pharmaceutical composition is 50 to 300 times the amount of the substance according to any one of (a) to (c) by mole.

11. The pharmaceutical composition according to any one of claims 1 to 10, wherein the pH is adjusted using a hydroxide of one or more metals selected from the group consisting of an alkali metal, an alkaline earth metal, and magnesium.

12. The pharmaceutical composition according to claim 11, wherein the hydroxide is one or more selected from the group consisting of potassium hydroxide, calcium hydroxide, sodium hydroxide, and magnesium hydroxide.

13. The pharmaceutical composition of claim 12, wherein the hydroxide is sodium hydroxide.

14. The pharmaceutical composition according to any one of claims 1 to 13, comprising the acid addition salt according to any one of (a) to (c), wherein the acid addition salt is a trifluoroacetate salt.

15. The pharmaceutical composition of any of claim 1 to 14, wherein the pharmaceutical composition is a freeze-dried product.

16. The pharmaceutical composition according to claim 15, wherein the amount of the substance according to any one of (a) to (c) based on 1 g of the freeze-dried product is 6.0 to 50.0 mg.

17. An injectable formulation prepared by dissolving a pharmaceutical composition according to any of claims 1 to 16 in distilled water for injection.

18. A method for producing a pharmaceutical composition, comprising the steps of:
1) dissolving a hydrate of an organic acid, a hydrate of a metal salt of an organic acid, and a sugar and/or a sugar alcohol in distilled water for injection to adjust a pH to 3.0 to 4.0;
2) cooling a liquid produced in the step 1) to 18°C or lower;
3) dissolving the substance according to any one of (a) to (c) of claim 1 in the liquid obtained in the step 2);
4) adjusting a pH of a liquid obtained in the step 3) to 2.5 to 4.0 with a hydroxide of one or more metals selected from the group consisting of an alkali metal, an alkaline earth metal, and magnesium; and
5) freeze-drying a liquid obtained in the step 4).

19. A method for producing a pharmaceutical composition, comprising the steps of:
1) dissolving citric acid hydrate, sodium citrate hydrate and sucrose in distilled water for injection and adjusting a pH to 3.0 to 4.0;
2) cooling a liquid produced in the step 1) to 18°C or lower;
3) dissolving the substance according to any one of (a) to (c) of claim 1 in a liquid obtained in the step 2);
4) adjusting a pH of the liquid obtained in the step 3) to 2.9 to 3.5 with sodium hydroxide; and
5) freeze-drying a liquid obtained in the step 4).

20. A pharmaceutical composition produced by the production method according to claim 18 or 19.
